(19) Europäisches Patentamt / European Patent Office / Office européen des brevets

(11) **EP 4 575 930 B1**

(12) **EUROPEAN PATENT SPECIFICATION**

(45) Date of publication and mention of the grant of the patent:
**21.01.2026 Bulletin 2026/04**

(21) Application number: **24220848.6**

(22) Date of filing: **18.12.2024**

(51) International Patent Classification (IPC):
**G06N 10/60** *(2022.01)*

(52) Cooperative Patent Classification (CPC):
**G06N 10/60**

(54) **IMPLEMENTATION OF DENSITY FUNCTIONAL THEORY ON QUANTUM COMPUTING THROUGH META GENERALIZED GRADIENT APPROXIMATION**

IMPLEMENTIERUNG EINER DICHTEFUNKTIONALTHEORIE AUF QUANTENBERECHNUNG DURCH META-VERALLGEMEINERTE GRADIENTENANNÄHERUNG

MISE EN OEUVRE D'UNE THÉORIE FONCTIONNELLE DE DENSITÉ SUR UN CALCUL QUANTIQUE PAR APPROXIMATION DE GRADIENT MÉTA GÉNÉRALISÉE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.12.2023 IN 202321087620**

(43) Date of publication of application:
**25.06.2025 Bulletin 2025/26**

(73) Proprietor: **Tata Consultancy Services Limited Maharashtra (IN)**

(72) Inventors:
• **MUKHERJEE, ANIRBAN**
 **400096 Mumbai, Maharashtra (IN)**
• **GOPAL, ANANTHAKRISHNA**
 **400096 Mumbai, Maharashtra (IN)**
• **BANERJEE, RITAM**
 **400096 Mumbai, Maharashtra (IN)**

(74) Representative: **Goddar, Heinz J.**
 **Boehmert & Boehmert**
 **Anwaltspartnerschaft mbB**
 **Pettenkoferstrasse 22**
 **80336 München (DE)**

(56) References cited:
• **ROSSMANNEK MAX ET AL: "Quantum HF/DFT-embedding algorithms for electronic structure calculations: Scaling up to complex molecular systems", THE JOURNAL OF CHEMICAL PHYSICS, vol. 154, no. 11, 21 March 2021 (2021-03-21), XP093141526, ISSN: 0021-9606, DOI: 10.1063/5.0029536**
• **TAEHEE KO ET AL: "Implementation of the Density-functional Theory on Quantum Computers with Linear Scaling with respect to the Number of Atoms", ARXIV.ORG, CORNELL UNIVERSITY LIBRARY, 201 OLIN LIBRARY CORNELL UNIVERSITY ITHACA, NY 14853, 13 July 2023 (2023-07-13), XP091563102**
• **ROBERT SCHADE ET AL: "Parallel Quantum Chemistry on Noisy Intermediate-Scale Quantum Computers", ARXIV.ORG, CORNELL UNIVERSITY LIBRARY, 201 OLIN LIBRARY CORNELL UNIVERSITY ITHACA, NY 14853, 11 August 2022 (2022-08-11), XP091292209**
• **ROSSMANNEK MAX ET AL: "Quantum Embedding Method for the Simulation of Strongly Correlated Systems on Quantum Computers", JOURNAL OF PHYSICAL CHEMISTRY LETTERS, vol. 14, no. 14, 6 February 2023 (2023-02-06), US, pages 1 - 8, XP093267643, ISSN: 1948-7185, DOI: 10.1021/acs.jpclett.3c00330**

- SAMBIT DAS ET AL: "DFT-FE 1.0: A massively parallel hybrid CPU-GPU density functional theory code using finite-element discretization", ARXIV.ORG, CORNELL UNIVERSITY LIBRARY, 201 OLIN LIBRARY CORNELL UNIVERSITY ITHACA, NY 14853, 15 March 2022 (2022-03-15), XP091190622

- RYAN PEDERSON ET AL: "Large scale quantum chemistry with Tensor Processing Units", ARXIV.ORG, CORNELL UNIVERSITY LIBRARY, 201 OLIN LIBRARY CORNELL UNIVERSITY ITHACA, NY 14853, 13 December 2022 (2022-12-13), XP091392282, DOI: 10.1021/ACS.JCTC.2C00876

**Description**

TECHNICAL FIELD

The disclosure herein generally relates to quantum computing, and, more particularly, to a method and system for implementation of density functional theory on quantum computing through meta generalized gradient approximation.

BACKGROUND

As quantum hardware and algorithms continue to develop, various industry sectors, particularly the pharmaceutical and material design domains, are applying quantum computation paradigm to their specific problems. For example, many chemical compounds are synthesized and predicted for practical use. Current software packages calculate the energetics and other physical properties of physical/chemical systems using a quantum mechanical (QM) electronic self-consistent field approach called the Kohn-Sham Density functional theory (KS-DFT). However, a computational time complexity bottleneck of the DFT approach has remained a cubic function of the number of electronic orbitals. This is also known as the cubic scaling wall bottleneck in DFT. Most industrially relevant chemical systems in Material sciences industry are bulk materials. One supercell made from a collection of neighboring unit cells involves around thousands of atoms which corresponds to several thousands of electronic orbitals even in the least correlated basis. Currently these chemical systems are treated approximately within the QM/Molecular Mechanical (MM) approach, where only a subsystem with strong quantum correlations is simulated using DFT and the rest is treated only via the MM technique. Identifying these subsystems requires doing prior Molecular mechanical and or Force field analysis and adds to additional overhead cost. The gap in applying this DFT technology to the complete system arises from the cubic scaling wall bottleneck i.e. for a 50000 electronic orbital system (corresponding to a supercell of the bulk material) computing even one KS-DFT step would require 0.001 secs on supercomputer FUGAKU having $R_{max}$ of 442 PETA Flops.

For all practical purposes, the DFT code should converge within 100 self-consistency steps therefore to simulate one large supercell on FUGAKU would require 0.1 seconds. For real world simulations one would require screening across several lakhs of molecular configurations in a supercell and that would require more than one day. The output electronic density computed from DFT needs to be in turn passed into Force-Field or Molecular mechanical modelling suites that then recomputes the geometric positioning of atoms and the DFT energies needs to be recomputed. Altogether that implies that for slightly larger system such calculations can enter several days of calculations even on the largest of the supercomputers. Similarly the chemical systems in Pharma industry that correspond to Protein drug or protein-protein systems involve more than thousands of atoms which corresponds to a several thousands of electronic orbitals. These chemical systems are treated approximately within the QM/Molecular Mechanical (MM) approach, only a subsystem is treated with strong quantum correlations and is simulated using DFT and the rest is treated only via the MM technique. Drug molecules in the pharma industry have more than 500 Molecular weight, screening across different drug molecules enters across several days of efforts. If time computational complexity of DFT for general chemical systems is reduced even nominally from cubic to quadratic or linear, then several days of calculations can be reduced to a few days or within a day. This would enable faster and more screening in a short duration of time, handling larger QM regions and reduce product discovery time drastically.

In conventional KS DFT, the computational complexity scales cubically to system size as a consequence of delocalized nature of wave functions which are the eigen solutions of the Kohn-Sham single particle Hamiltonian. For scaling the DFT calculations to large systems, there have been attempts to develop a technique which scales linearly with system size. One such technique is Order-N Electronic Total Energy Package (ONETEP) which uses a basis of non-orthogonal generalized Wannier functions (NGWFs) expressed in terms of periodic cardinal sine (psinc) functions, which are in turn equivalent to a basis of plane-waves. ONETEP, therefore combines benefits of linear scaling with a level of accuracy and variational bounds comparable to traditional cubic-scaling plane-wave approaches. However, ONETEP is primarily designed for periodic systems restricting its application to certain materials and structures. Further, in current scenario, there have efforts in speeding up the DFT calculations. The conventions method for speeding up the DFT calculations include Exascale computing using central processing unit (CPU), general processing unit (GPU), message passing interface (MPI), Multiprocessing, and/or the like. However, these have memory and processing limitations. There have been attempts made to implement DFT on a combination of classical and quantum processors. One such approach implements DFT on a classical processor and optimizes the DFT results on a quantum processor. However, the complex calculations are still performed on the classical processor thereby bottlenecks in DFT calculations are still challenging to be overcome.

Rossmannek Max et al: "Quantum HF/DFT-embedding algorithms for electronic structure calculations: Scaling up to complex molecular systems" discloses two embedding schemes for quantum electronic structure algorithms based on HF and Kohn-Sham (KS) DFT Molecular Orbitals (MOs). The subsystem solved by means of the quantum approach [such as quantum Unitary Coupled Cluster Singles and Doubles (q-UCCSD)] is embedded in the potential generated by the environment (i.e., the remaining electrons), which is computed within the HF or DFT framework. The solutions are based on the Range-Separation (RS) technique for the two-electron integrals, which allows for a rigorous partitioning of the problem into a subsystem (i.e., the AS) and its environment. Furthermore, in the case of the DFT embedding scheme the algorithm is extended to include the self-consistent optimization of the embedding potential, leading to more accurate energies and densities (Abstract).

Ko Taehee et al: "Implementation of the Density-functional Theory on Quantum Computers with Linear Scaling with respect to the Number of Atoms" presents a quantum algorithm that has a linear scaling with respect to the number of atoms, which is much smaller than the number of electrons. The quantum algorithm leverages the quantum singular value transformation (QSVT) to generate a quantum circuit to encode the density-matrix, and an estimation method for computing the output electron density. In addition, a randomized block coordinate fixed-point method is disclosed which accelerates the self-consistent field calculations by reducing the number of components of the electron density that needs to be estimated (Abstract).

Robert Schade et al: "Parallel Quantum Chemistry on Noisy Intermediate-Scale Quantum Computers" presents a parallel hybrid quantum-classical algorithm for the solution of the quantum-chemical ground-state energy problem on gate-based quantum computers. This approach is based on the reduced density-matrix functional theory (RDMFT) formulation of the electronic structure problem. For that purpose, the density-matrix functional of the full system is decomposed into an indirectly coupled sum of density-matrix functionals for all its subsystems using the adaptive cluster approximation to RDMFT. The approximations involved in the decomposition and the adaptive cluster approximation itself can be systematically converged to the exact result. The solutions for the density-matrix functionals of the effective subsystems involves a constrained minimization over many-particle states that are approximated by parametrized trial states on the quantum computer similarly to the variational quantum eigensolver. The independence of the density-matrix functionals of the effective subsystems introduces a new level of parallelization and allows for the computational treatment of much larger molecules on a quantum computer with a given qubit count. In addition, for the proposed algorithm techniques are presented to reduce the qubit count, the number of quantum programs, as well as its depth (Abstract).

Rossmannek Max et al: "Quantum Embedding Method for the Simulation of Strongly Correlated Systems on Quantum Computers" employs projection-based embedding method for combining the variational quantum eigensolver (VQE) algorithm, although not limited to, with density functional theory (DFT). The developed VQE-in-DFT method is then implemented efficiently on a real quantum device and employed for simulating the triple bond breaking process in butyronitrile (Abstract).

Sambit Das et al: "DFT-FE 1.0: A massively parallel hybrid CPU-GPU density functional theory code using finite-element discretization" presents DFT-FE 1.0 to conduct fast and accurate large-scale density functional theory (DFT) calculations (reaching ~ 100, 000 electrons) on both many-core CPU and hybrid CPU-GPU computing architectures. This work involves improvements in the real-space formulation-via an improved treatment of the electrostatic interactions-that substantially enhances the computational efficiency-as well high performance computing aspects, including the GPU acceleration of all the key compute kernels in DFT-FE (Abstract).

Ryan Pederson et al: "Large scale quantum chemistry with Tensor Processing Units" discloses Tensor Processing Units (TPUs) as quantum chemistry supercomputers by accelerating the $O(N3)$ computational bottleneck of DFT approaches which use an auxiliary single particle kinetic energy approximation, such as Kohn Sham (KS) and generalized KS (gKS) DFT, where gKS admits hybrid DFT functionals (Abstract).

SUMMARY

Embodiments of the present disclosure present technological improvements as solutions to one or more of the above-mentioned technical problems recognized by the inventors in conventional systems. In one embodiment, a method for implementation of density functional theory on quantum computing through meta generalized gradient approximation is provided. The invention is set out in the appended set of claims 1-4.

In another aspect, a system for implementation of density functional theory on quantum computing through meta generalized gradient approximation is provided. The invention is set out in the appended set of claims 5-8.

In yet another aspect, a computer program product including a non-transitory computer-readable medium having embodied therein a computer program implementation of density functional theory on quantum computing through meta generalized gradient approximation is provided. The invention is set out in the appended set of claims 9-12.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a functional block diagram of a system for implementation of density functional theory on quantum computing through meta generalized gradient approximation, in accordance with some embodiments of the present disclosure.

FIGS. 2A through 2E, collectively referred as FIG. 2 is an exemplary flow diagram illustrating for implementation of density functional theory on quantum computing through meta generalized gradient approximation, implemented by the system of FIG. 1, in accordance with some embodiments of the present disclosure.

FIG. 3 illustrates an alternative representation of the flow diagram of FIG. 2, in accordance with some embodiments of the present disclosure.

FIG. 4 illustrates a quantum circuit used to compute a direct matrix in the method illustrated in FIG. 2, in accordance with some embodiments of the present disclosure.

FIG. 5 illustrates a first quantum circuit block that encodes a collocation matrix in the method illustrated in FIG. 2, in accordance with some embodiments of the present disclosure.

FIGS. 6A through 6F illustrates quantum circuit blocks that encode different components of the Hessian of the collocation matrix in the method illustrated in FIG. 2, in accordance with some embodiments of the present disclosure.

FIG. 7 illustrates a quantum circuit comprising the second hessian quantum circuit block, the third hessian quantum circuit block, and the fourth hessian quantum circuit block that encode the first, second and third components of the hessian of electronic density respectively in the method illustrated in FIG. 2, in accordance with some embodiments of the present disclosure.

FIG. 8 illustrates a fifth quantum circuit block that encodes Hessian of the electronic density in the method illustrated in FIG. 2, in accordance with some embodiments of the present disclosure.

FIG. 9 illustrates the eight quantum circuit block that encodes the correlation exchange matrix in the method illustrated in FIG. 2, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

As quantum hardware and algorithms continue to develop, various industry sectors, particularly the pharmaceutical and material design domains, are applying quantum computation paradigm to their specific problems. For example, many chemical compounds are synthesized and predicted for practical use. Current software packages calculate the energetics and other physical properties of physical/chemical systems using a quantum mechanical (QM) electronic self-consistent field approach called the Kohn-Sham Density functional theory (KS-DFT). However, the computational time complexity bottleneck of the DFT approach has remained a cubic function of the number of electronic orbitals. This is also known as the cubic scaling wall bottleneck in DFT. Further, conventional methods implement the DFT simulations on classical processors such as CPU, GPU etc. which is time consuming. Some state of the art techniques implement DFT on a combination of classical and quantum processors. However, the complex calculations are still performed on a classical processor which doesn't overcome the bottlenecks in DFT calculations.

The present disclosure addresses the unresolved problem of computational time complexity bottleneck of the DFT in the conventional methods by providing a system and method for implementation of density functional theory on quantum computing through meta generalized gradient approximation. In the present disclosure, diagonalization problem is mapped to a sequence of quadratic least squares problem and solved as a quantum linear systems problem using implementation of density functional theory on quantum computing through meta generalized gradient approximation. More specifically, the present disclosure provides a quantum circuit for computing meta generalized gradient approximation (meta GGA) density functional computations efficiently on a quantum processor. Initially, atomic coordinates of each atom in a chemical compound whose one or more properties must be extracted is received. Electron integrals, a core Hamiltonian, and a collocation matrix is computed from the atomic coordinates. The core Hamiltonian is diagonalized to obtain a density matrix of the chemical compound which is further updated iteratively until a convergence criteria is satisfied, using the meta GGA. At each iteration, a direct matrix is computed from the density matrix, a correlation exchange matrix is computed from the direct matrix and the collocation matrix, a Fock matrix is computed by adding the direct matrix and the correlation exchange matrix and the Fock matrix is diagonalized to obtain updated density matrix which is used in subsequent iteration. This is repeated until norm of a difference between the updated density matrix at a current iteration and the density matrix at a previous iteration is lesser than a predefined threshold to obtain a final density matrix of the chemical compound which can be used to extract the one or more properties of the chemical compound.

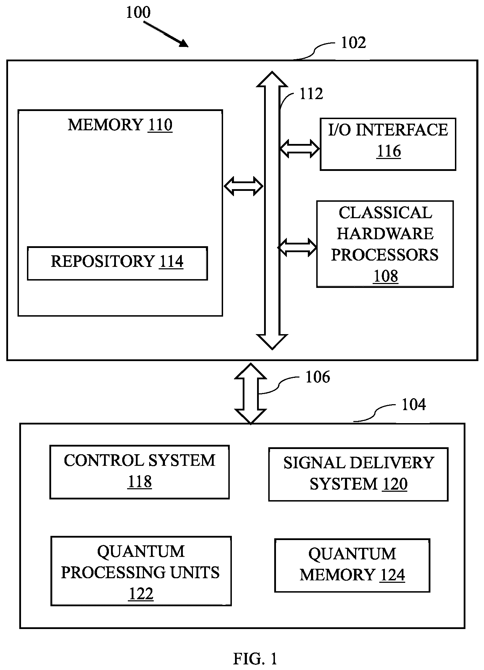

Referring now to the drawings, and more particularly to FIGS. 1 through 5. where similar reference characters denote corresponding features consistently throughout the figures, there are shown preferred embodiments and these embodiments are described in the context of the following exemplary system and/or method.

FIG. 1 is a functional block diagram of a system 100 for implementing density functional theory on quantum processors through local density approximation for simulating chemical compounds, in accordance with some embodiments of the present disclosure. The system 100 includes a classical computing system 102, a quantum computing system 104 and a communication interface 106.

The classical computing system 102 comprises one or more classical hardware processors 108, at least one memory such as a memory 110, and an I/O interface 116. The one or more classical hardware processors 108, the memory 110, and the Input /Output (I/O) interface 116 may be coupled by a system bus such as a system bus 112 or a similar mechanism. In an embodiment, the one or more classical hardware processors 108 can be one or more hardware processors. The one or more classical hardware processors and the hardware processors are interchangeably used throughout the document. Similarly, the classical computing system 102 is a normal computing system.

The I/O interface 116 may include a variety of software and hardware interfaces, for example, a web interface, a graphical user interface, and the like., for example, interfaces for peripheral device(s), such as a keyboard, a mouse, an external memory, a printer and the like. Further, the I/O interface 116 may enable the system 100 to communicate with other devices, such as web servers, and external databases. The I/O interface 116 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 116 may include one or more ports for connecting several computing systems with one another or to another server computer.

The one or more hardware processors 108 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 108 is configured to fetch and execute computer-readable instructions stored in the memory 110.

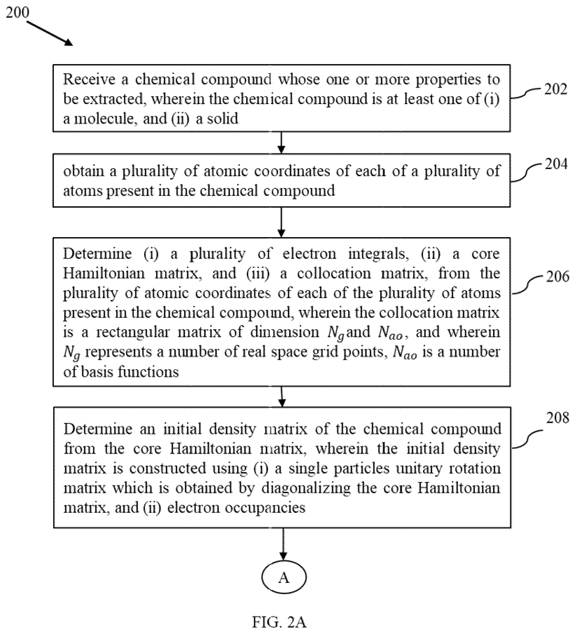

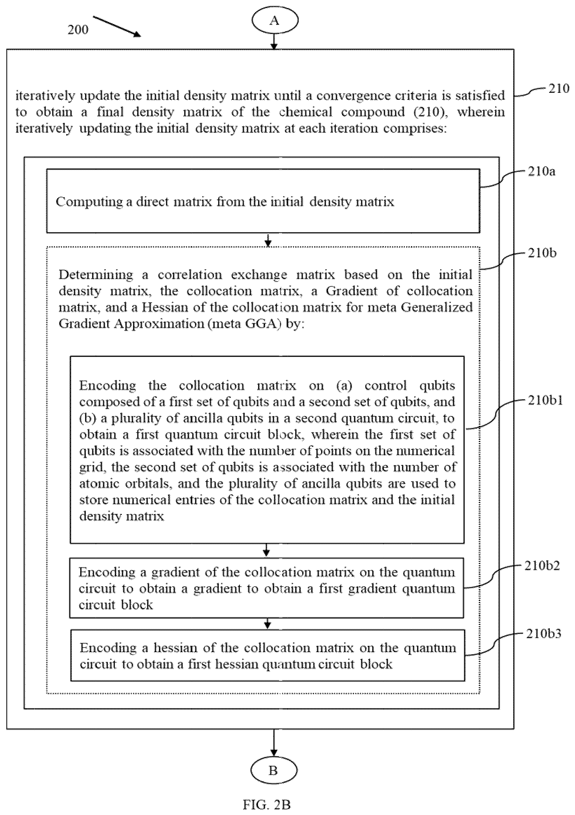

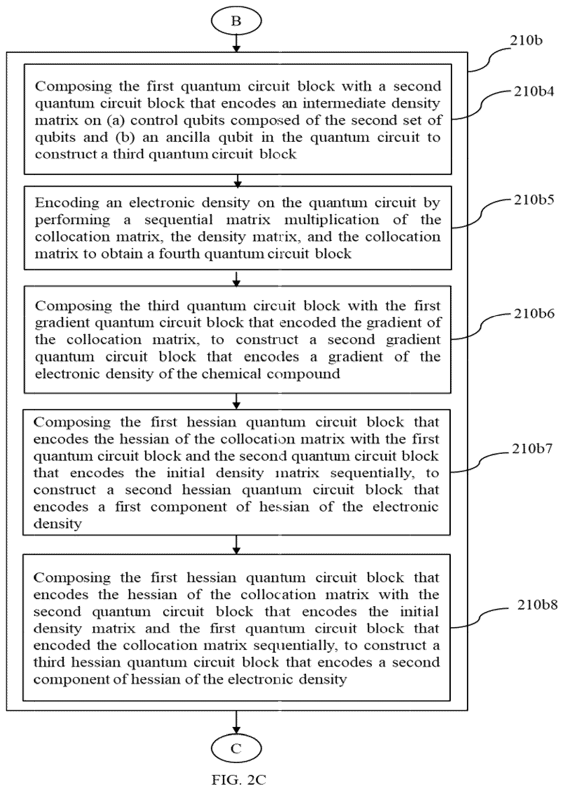

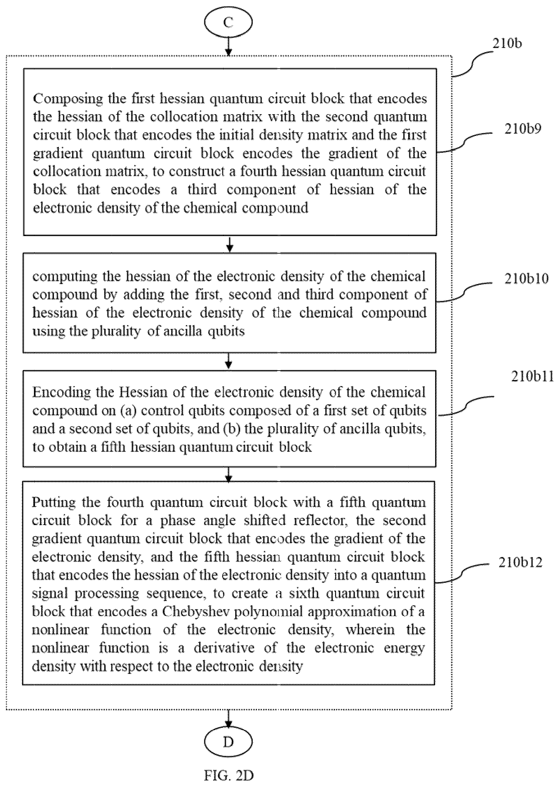

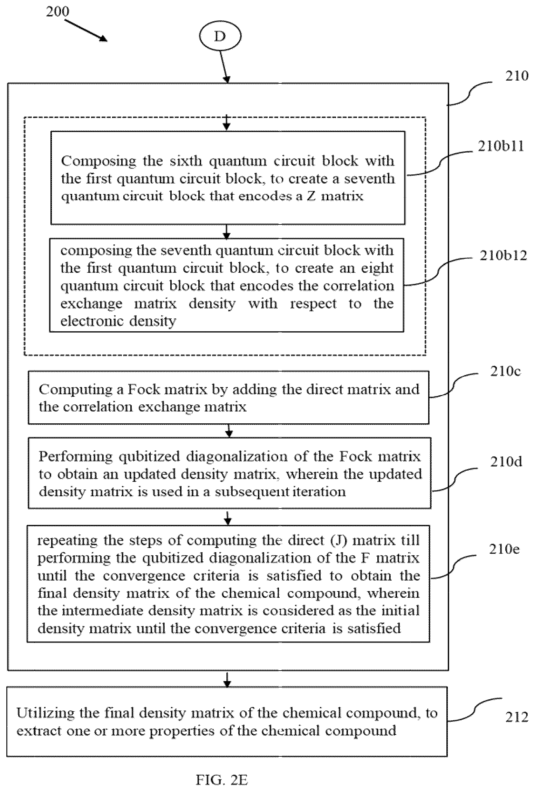

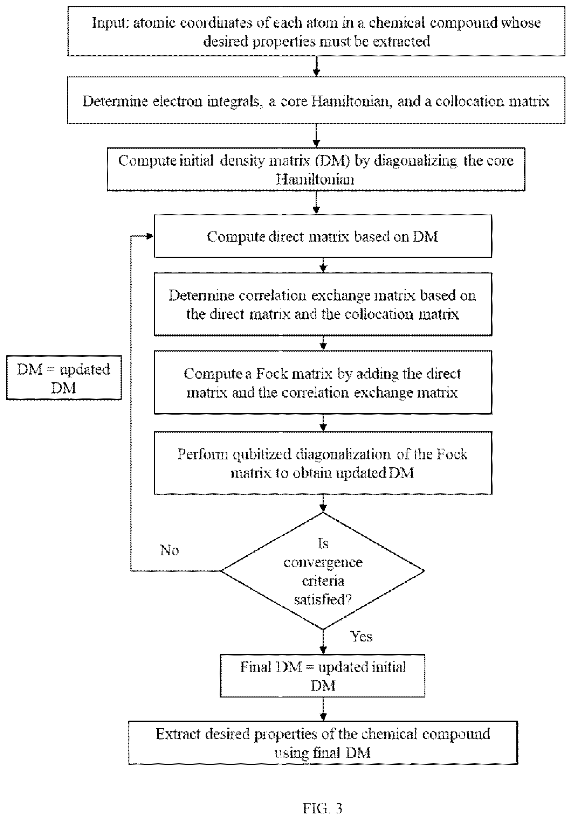

The memory 110 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 110 includes a data repository 114. The data repository (or repository) 114 may include a plurality of abstracted piece of code for refinement and data that is processed, received, or generated as a result of the execution of the method illustrated in FIGS. 2 and 3. Although the data repository 114 is shown internal to the system 100, it should be noted that, in alternate embodiments, the data repository 114 can also be implemented external to the system 100, where the data repository 114 may be stored within a database (repository 114) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

The quantum computing system 104 shown in FIG. 1 includes a control system 118, a signal delivery system 120, a plurality of quantum processors (alternatively referred as Quantum Processing Units (QPUs)) 122, and a quantum memory 124. The plurality of quantum processors 122 are unentangled and hence alternatively referred as the plurality of unentangled quantum processors 122. The quantum computing system 104 may include additional or different features, and the components of a quantum computing system 104 may operate as described with respect to FIG. 1 or in alternative manner.

In an embodiment, the quantum computing system 104 can perform quantum computational tasks (such as, for example, quantum simulations or other quantum computational tasks) by executing quantum algorithms. In some implementations, the quantum computing system 104 can perform quantum computation by storing and manipulating information within individual quantum states of a composite quantum system. For example, Qubits (i.e., Quantum bits) can be stored in and represented by an effective two-level sub-manifold of a quantum coherent physical system in the plurality of unentangled quantum processors 122. In an embodiment, the quantum computing system 104 can operate using gate-based models for quantum computing. For example, the Qubits can be initialized in an initial state, and a quantum logic circuit comprised of a series of quantum logic gates can be applied to transform the qubits and extract measurements representing the output of the quantum computation.

The plurality of unentangled quantum processors 122 shown in FIG. 1 may be implemented, for example, as a superconducting quantum integrated circuit that includes Qubit devices. The Qubit devices may be used to store and process quantum information, for example, by operating as ancilla Qubits, data Qubits or other types of Qubits in a quantum algorithm. Coupler devices in the superconducting quantum integrated circuit may be used to perform quantum logic operations on single qubits or conditional quantum logic operations on multiple qubits. In some instances, the conditional quantum logic can be performed in a manner that allows large-scale entanglement within the plurality of unentangled quantum processors 122. Control signals may be delivered to the superconducting quantum integrated circuit, for example, to manipulate the quantum states of individual Qubits and the joint states of multiple Qubits. In some instances, information can be read from the superconducting quantum integrated circuit by measuring the quantum states of the qubit devices. The plurality of unentangled quantum processors 122 may be implemented using another type of physical system.

The plurality of unentangled quantum processors 122, and in some cases all or part of the signal delivery system 120, can be maintained in a controlled cryogenic environment. The environment can be provided, for example, by shielding equipment, cryogenic equipment, and other types of environmental control systems. In some examples, the components in the plurality of unentangled quantum processors 122 operate in a cryogenic temperature regime and are subject to very low electromagnetic and thermal noise. For example, magnetic shielding can be used to shield the system components from stray magnetic fields, optical shielding can be used to shield the system components from optical noise, thermal shielding and cryogenic equipment can be used to maintain the system components at controlled temperature, etc.

In an embodiment, the signal delivery system 120 provides communication between the control system 118 and the plurality of unentangled quantum processors 122. For example, the signal delivery system 120 can receive control signals from the control system 118 and deliver the control signals to the plurality of unentangled quantum processors 122. In some instances, the signal delivery system 120 performs preprocessing, signal conditioning, or other operations to the control signals before delivering them to the plurality of unentangled quantum processors 122. In an embodiment, the signal delivery system 120 includes connectors or other hardware elements that transfer signals between the plurality of unentangled quantum processors 122 and the control system 118. For example, the connection hardware can include signal lines, signal processing hardware, filters, feedthrough devices (e.g., light-tight feedthroughs, etc.), and other types of components. In some implementations, the connection hardware can span multiple different temperature and noise regimes. For example, the connection hardware can include a series of temperature stages that decrease between a higher temperature regime (e.g., at the control system 118) and a lower temperature regime (e.g., at the plurality of unentangled quantum processors 122).

In an embodiment, the control system 118 controls operation of the plurality of unentangled quantum processors 122. For example, the control system 118 may include data processors, signal generators, interface components and other types of systems or subsystems. Components of the example control system 118 may operate in a room temperature regime, an intermediate temperature regime, or both. For example, the control system 118 can be configured to operate at much higher temperatures and be subject to much higher levels of noise than are present in the environment of the plurality of unentangled quantum processors 122.

In some embodiments, the control system 118 includes a classical computing system that executes software to compile instructions for the plurality of unentangled quantum processors 122. For example, the control system 118 may decompose a quantum logic circuit or quantum computing program into discrete control operations or sets of control operations that can be executed by the hardware in the plurality of unentangled quantum processors 122. In some examples, the control system 118 applies a quantum logic circuit by generating signals that cause the Qubit devices and other devices in the plurality of unentangled quantum processors 122 to execute operations. For instance, the operations may correspond to single-Qubit gates, two-Qubit gates, Qubit measurements, etc. The control system 118 can generate control signals that are communicated to the plurality of unentangled quantum processors 122 by the signal delivery system 120, and the devices in the plurality of unentangled quantum processors 122 can execute the operations in response to the control signals.

In some other embodiments, the control system 118 includes one or more classical computers or classical computing components that produce a control sequence, for instance, based on a quantum computer program to be executed. For example, a classical processor may convert a quantum computer program to an instruction set for the native gate set or architecture of the plurality of unentangled quantum processors 122. In some cases, the control system 118 includes a microwave signal source (e.g., an arbitrary waveform generator), a bias signal source (e.g., a direct current source) and other components that generate control signals to be delivered to the plurality of unentangled quantum processors 122. The control signals may be generated based on a control sequence provided, for instance, by a classical processor in the control system 118. The example control system 118 may include conversion hardware that digitizes response signals received from the plurality of unentangled quantum processors 122. The digitized response signals may be provided, for example, to a classical processor in the control system 118.

In some embodiments, the quantum computer system 104 includes multiple quantum information processors that operate as respective unentangled quantum processors 122. In some cases, each quantum processor can operate independently. For instance, the quantum computer system 104 may be configured to operate according to a distributed quantum computation model, or the quantum computer system 104 may utilize multiple quantum processors in another manner. In some implementations, the quantum computer system 104 includes multiple control systems, and each quantum processor may be controlled by a dedicated control system. In some implementations, a single control system can control multiple quantum processors. For instance, the control system 118 may include multiple domains that each control a respective quantum processor. In some instances, the quantum computing system 104 uses multiple quantum processors to execute multiple unentangled quantum computations (e.g., multiple Variational Quantum Eigen solver (VQE)) that collectively simulate a single quantum mechanical system.

In an embodiment, the quantum memory 124 is a quantum-mechanical version of classical computer memory 110. The classical computer memory 100 stores information such as binary states and the quantum memory 124 stores a quantum state for later retrieval. These states hold useful computational information known as Qubits. In an embodiment, the communication interface 106 which connects the classical computing system 102 and the quantum computing system 104 is a high speed digital interface.

FIGS. 2A through 2E, collectively referred as FIG. 2 is an exemplary flow diagram illustrating a method 200 for implementation of density functional theory on quantum computing through meta generalized gradient approximation, implemented by the system of FIG. 1, in accordance with some embodiments of the present disclosure. In an embodiment, the system 100 is configured to store instructions for execution of steps of the method 200 by the one or more hardware processors 108 and the plurality of unentangled quantum processors 122. FIG. 3 illustrates an alternative representation of the flow diagram of FIG. 2, in accordance with some embodiments of the present disclosure.

The steps of the method 200 of the present disclosure will now be explained with reference to the components or blocks of the system 100 as depicted in FIG. 1 and the steps of flow diagram as depicted in FIGS. 2 and 3. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

Now referring to FIG. 2, at step 202 of the method 200, one or more classical hardware processors are configured to receive a chemical compound whose one or more properties are to be extracted. The chemical compound is at least one of (i) a molecule, and (ii) a solid (202). In the context of the present disclosure, the expression 'solid' refers to a periodic arrangements of atoms. For example, in pharma industry, the chemical compound maybe a drug lead and its conformations whose physical properties must be analyzed to determine feasibility of synthesizing the drug from the drug lead. The one or more properties of drug lead includes one or more physical and one or more chemical properties such as highest occupied molecular orbital and lowest unoccupied molecular (HOMO-LUMO) gap in solvent medium, acid-base dissociation constant (*pKa*) of a drug, dipole moment, partial charges, and/or the like.

Further, at step 204 of the method 200, the one or more classical hardware processors are configured to obtain a plurality of atomic coordinates of each of a plurality of atoms present in the chemical compound. For example, for drug paracetamol, a list of the plurality of atomic coordinates (*X, Y, Z*) with a corresponding atomic symbol is obtained as: (2.8660 -2.5950 0.0000 *O*), ( 4.5981 1.4050 0.0000 *O*), (2.8660 1.4050 0.0000 *N*), (2.8660 0.4050 0.0000 *C*), (1.4631 0.2150 0.0000 *H*), and/or the like.

Further, at step 206 of the method 200, the one or more classical hardware processors are configured to determine (i) a plurality of electron integrals, (ii) a core Hamiltonian matrix, and (iii) a collocation matrix, from the plurality of atomic coordinates of each of the plurality of atoms present in the chemical compound. The collocation matrix is a rectangular matrix of dimension $N_g$ and $N_{ao}$, where $N_g$ represents a number of real space grid points, $N_{ao}$ is a number of basis functions. The collocation matrix comprises a plurality of basis functions of a plurality of atomic orbitals and a plurality of points on numerical grid. Each of the plurality of basis functions is a Gaussian wave function centered around the plurality of atomic coordinates. Thus, the plurality of basis functions are represented as Gaussians functions as a function of the plurality atomic coordinates of the plurality of atoms and an angular and spin momentum. The collocation matrix is mathematically represented using equation (1) below:

$$\Phi = \begin{pmatrix} \phi_1(x_1, y_1, z_1), \ldots \ldots \ldots \phi_1\left(x_{N_g}, y_{N_g}, z_{N_g}\right) \\ \vdots \\ \phi_{Nao}(x_1, y_1, z_1), \ldots \ldots \ldots \phi_{Nao}\left(x_{N_g}, y_{N_g}, z_{N_g}\right) \end{pmatrix} (1)$$

The plurality of basis functions are used to compute the plurality of electron integrals. The plurality of electron integrals include one-electron integrals and two-electron integrals which are further bifurcated as three centered and two centered electron integrals. Thus, the plurality of electron integrals include (a) 3 center 2 electron integrals, b) 2 center 2 electron integrals and c) 2 center 1 electron integrals. The 3 center 2 electron integrals and 2 center 2 electron integrals describe a Coulomb repulsion integral that are computed in atomic orbital basis and can be represented in either spherical or cartesian coordinates. The 2 center 1 electron integrals describes an overlap between the basis states. The plurality of electron integrals are determined using tools such as LIBCINT, Python-based Simulations of Chemistry Framework (PySCF), NorthWest computational Chemistry (NWchem), and/or the like. The core Hamiltonian matrix which is a $N_b \times N_b$

matrix is determined using the Kohn-Sham (KS) hybrid Density Functional Theory (DFT) process which is initiated from a set of $N_b$ basis functions $\mathbb{B} = \{\phi_\mu(r)\}_{\mu=1}^{N_b}$ to compute the core Hamiltonian matrix $h$. $h$ constitutes free particle energy of electrons and one-body nuclear potential arising from electron-nucleus interactions, both these terms are independent of electronic density. Therefore $h$ needs to be computed while initiating a self-consistent field (SCF) procedure.

Further, at step 208 of the method 200, the one or more classical hardware processors and the plurality of unentangled QPUs are configured to determine an initial density matrix of the chemical compound from the core Hamiltonian matrix. The initial density matrix is constructed using (i) a single particles unitary rotation matrix which is obtained by diagonalizing the core Hamiltonian matrix, and (ii) electron occupancies (e.g., refer Indian patent application IN 20232106141 titled 'Method And System For Reducing Time Complexity Of Density Functional Theory Calculations With Qubitized Diagonalization').

Once the density matrix is determined, at step 210 of the method 200, the one or more classical hardware processors and the plurality of unentangled QPUs are configured to iteratively update the initial density matrix until a convergence criteria is satisfied to obtain a final density matrix of the chemical compound. The convergence criteria is satisfied when a norm of a difference between an intermediate density matrix at a current iteration and the initial density matrix at a previous iteration is lesser than a predefined threshold. For iteratively updating the initial density matrix at each iteration, steps 210a to 210e are performed at each iteration to update the initial density matrix. In an embodiment, the step 210a is better understood by way of following description as exemplary explanation.

In the Kohn-Sham (KS) hybrid Density Functional Theory (DFT) process, the direct ($J$) matrix and an exact-exchange ($K$) matrix are electronic density dependent terms which are computed from an Electronic Repulsion Integral (ERI) tensor. Further, in the Kohn-Sham (KS) hybrid Density Functional Theory (DFT) process, a correlation exchange potential or a correlation exchange matrix ($V^{xc}$) is computed from an electronic density, its gradient and/or a Kinetic energy density. Together $J$, $K$ and $V^{xc}$ make a hybrid Kohn-Sham Fock Matrix functional F as given by equation (2)

$$F[D] = h + J[D] - cK[D] + V^{xc}[D] \quad (2)$$

In equation (2), $D$ is a one-particle density matrix discretized in a basis of $\{\phi_\mu\}$ and $c \in \mathbb{R}^+$ is a modulation for the exact-exchange term. The terms $J$, $K$ can be computed from an atomic orbital (AO)-integral approach using the ERI tensor represented as $\langle ij|kl \rangle$ and $D$ as shown below in equations (3) and (4), respectively.

$$J_{ij} = \sum_{kl}\langle ij|kl \rangle D_{kl} \quad (3)$$

$$K_{ij} = \sum_{kl}\langle ik|jl \rangle D_{kl} \quad (4)$$

In equations (3) and (4), the ERI tensor $\langle ij|kl \rangle$ is obtained by integrating space of basis functions in $\mathbb{R}^3$ according to equation (5) shown below:

$$\langle ij|kl \rangle = \int d^3r\, d^3r' \phi_i(r)\phi_j(r)\frac{1}{|r-r'|}\phi_k(r')\phi_l(r'). \quad (5)$$

The correlation-exchange potential $V^{xc}$ in the discretized basis $\{\phi_i\}$ is computed from equation (6) shown below, where the basis functions are defined according to equation (7).

$$V_{ij}^{xc} = \int_{R^3} d^3r\, \phi_i(r)\frac{\delta E^{xc}[\rho(r)]}{\delta\rho(r)}\phi_j(r). \quad (6)$$

$$\phi_a(r) = (x - A_x)^{a_x}\big(\mathcal{Y} - A_y\big)^{a_y}(z - A_z)^{a_z}\exp(-\vartheta(x - A_x)^2)\exp\Big(-\vartheta\big(\mathcal{Y} - A_y\big)^2\Big)\exp(-\vartheta(z - A_z)^2) \quad (7)$$

In equation 6, $A_x$, $A_y$, $A_z$ are nuclear coordinate locations of the Gaussian functions, $a = (a_x, a_y, a_z)$ are the integer cartesian quanta and $\vartheta$ is the cartesian Gaussian exponent. In equation (6), $E^{xc}$ is an exchange energy functional evaluated for the electronic density $r$ and its form depends on a DFT method implemented using a plurality of classes of Density Functional Theory (DFT) protocols. The plurality of classes of DFT protocols comprise local density approximation (LDA), generalized gradient approximation (GGA), meta GGA, hybrid DFT and double hybrid DFT. In the context of the present disclosure, the DFT protocol used is meta Generalized Gradient Approximation (meta GGA). The ERI tensor is further represented using a Cholesky decomposition representation as given by equation (8) below.

$$\langle ij|kl\rangle = \sum_P L_{ij}^P L_{kl}^P \qquad (8)$$

$L^P$ represents Cholesky matrices that can be obtained from a Density-Fitting (DF) or a resolution of identity (RI) method as in equation (9).

$$L_{ij}^P = \frac{1}{\sqrt{w_P}} \sum_Q u_{QP} \langle ij|Q\rangle. \qquad (9)$$

In equation (9), $\langle ij|Q\rangle$ are 3-center 2-electron integrals represented in terms of the auxiliary basis functions $\{\chi_P(r)\}_{P=1}^{Naux}$, and $u_{QP}$, $w_P$ are obtained from the spectral decomposition of 2-center 2-electron integrals $V_{PQ} = \sum_R u_{PR} w_R u_{QR}^*$ represented in the auxiliary basis functions. The 3-center 2-electron integrals are given by equation (10) and the two center $V_{PQ} = \langle P|Q\rangle$ represents two center two electron integrals which are given by equation (11).

$$\langle ij|P\rangle = \int d^3r d^3r' \frac{\phi_i(r)\phi_j(r)\Lambda_P(r')}{|r-r'|} \qquad (10)$$

$$\langle P|Q\rangle = \int d^3r \frac{\Lambda_P(r)\Lambda_Q(r)}{|r-r'|} \qquad (11)$$

The $J$ and $K$ matrix elements are computed using the RI approach as in equation (12).

$$J_{ij} = \sum_P L_{ij}^P \sum_{kl} L_{kl}^P D_{kl}, K_{ij} = \sum_{Pl} L_{il}^P \sum_k L_{jk}^P D_{kl} \qquad (12)$$

When the KS-DFT is classically computed, the complexity of computing $J$ and $K$ is $O(pN^2)$ and this complexity resides in computing $K$. Computing $h$ and $V^{xc}$ has lower complexity of $O(N^2)$.

For executing any of the plurality of classes of Density Functional Theory (DFT) protocols, next step is to solve generalized eigenvalue problem for the Fock matrix accounting for an overlap between basis functions $S_{ij} = \langle \phi_i|\phi_j\rangle$ and an intermediate density matrix $D^{(l)}$ of a current iteration as given by equation (13).

$$F[D^{(l)}]C^{(l+1)} = SC^{(l+1)}\hat{E}^{(l+1)} \qquad (13)$$

In equation (13), the one particle density matrix or intermediate density matrix $D^{(l)} = C^{(l)}WC^{(l)\dagger}$, where an occupancy matrix $W_{ij} = \theta(\mu - E_i)(\delta_{ij}$ fills up $N_e/2$ lowest energy levels below a chemical potential $\mu$.

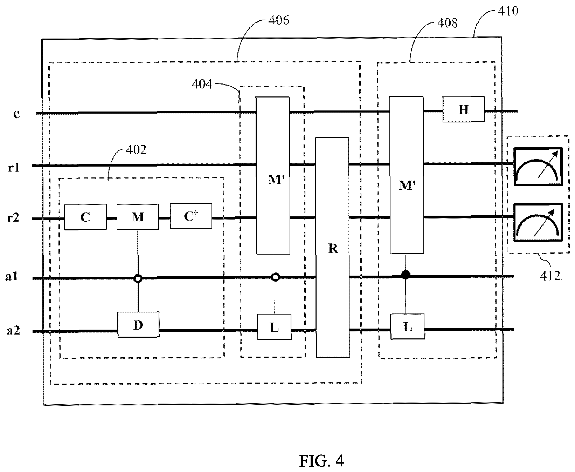

In the present disclosure, at step 210a, a direct matrix (alternatively referred to as $J$ matrix or Coulomb matrix) is computed from the initial density matrix using a quantum circuit as illustrated in FIG. 4. The quantum circuit comprises a first set of qubits, a second set of qubits and a plurality of ancilla qubits. The first set of qubits is associated with number of auxiliary basis functions in density fitting approximation of the plurality electron integrals (more particularly the 2 centered 2 electron integral), and the second set of qubits is associated with number of basis functions. In FIG. 4, 'c' represents the first set of qubits, 'r1, r2' represent the second set of qubits and 'a1, a2' represent the plurality of ancilla qubits. In an embodiment, the quantum circuit is mathematically represented as: $log\,N_{aux} + 2log\,N_{ao} + 2$ qubits. Initially, the initial density matrix is encoded (alternatively referred as loaded or block encoded) on the second set of qubits in the quantum circuit to form a first quantum circuit component (represented by block 402 in FIG. 4). As shown in block 402, the control is loaded on $r2$ and data elements of the initial density matrix are loaded on ancilla qubit $a2$. The density matrix is loaded using its Eigen decomposition representation that involves: i) $^{Nao}C_2$ Givens rotations on $log\,N_{ao}$ qubits, ii) Then block encoding a diagonal matrix of 1's in initial $N_e/2$ diagonal blocks and zeros in the rest using $2log\,N_{ao}$ qubits, iii) Another set of $^{Nao}C_2$ Givens rotation with conjugate angles on $N_e/2$ diagonals. This is mathematically represented by equation (14).

$$O(D) = I_2^{\otimes m} \otimes \left[ (C \otimes I_2^{\otimes n} \otimes I_2^{a_1})(\sum_k |k,k\rangle\langle k,k| \otimes (\theta(\mu - E_k)I_{a1} + (1 - \theta(\mu - E_k))Y_{a1})(C^\dagger \otimes I_2^{\otimes n} \otimes I_2^{a_1}) \right] \otimes I_{a2} \ldots\ldots (14)$$

The readouts from the encoding of the initial density matrix are obtained by equation (15).

$$\langle P, i, 0,0,0 | O(D) | P, j, 0,0,0 \rangle = \sum_{k=1}^{N_e/2} C_{ik} C_{kj}^* = D_{ij}, \text{ where } N_e = 2 \sum_{k=1}^{N} \theta(\mu - E_k)$$

$$\ldots (15)$$

Once the initial density matrix is encoded, a Cholesky tensor is encoded on the first quantum circuit to form a first Cholesky circuit component (represented by block 404 in FIG. 4). The Cholesky tensor is obtained from one or more electron integrals among the plurality of electron integrals, particularly, 3-center 2-electron and 2-center 2-electron integrals. As shown in the block 404, the controls are encoded on the $log\ N_{aux}$ + $2log\ N_{ao}$ qubits and the data is loaded on the ancilla qubits. This is mathematically represented by equation 15.

$$V_L = \sum_{c=0,r_1=0,r_2=0}^{Naux-1,Nao-1} \left[ |c, r_1, r_2, 0\rangle\langle c, r_1, r_2, 0| \otimes I_2 + |c, r_1, r_2, 1\rangle\langle c, r, 1| \otimes \left( L_{r_1,r_2}^{c'} I + i\sqrt{1 - \left(L_{r_1,r_2}^{c'}\right)^2} Y \right) \right] \ldots\ldots (15)$$

Further, the first quantum circuit component is composed with the first Cholesky circuit component and a diffusion operator to create a second quantum circuit component that processes the density matrix to generate an intermediate state vector as illustrated by block 406. The diffusion operator $R$ is given by equation 16.

$$R = \sum_{kl} \left[ 2\frac{|k,+,0\rangle\langle l,+,0|}{Naux} \otimes I_2^{\otimes 2n} - I \right] \ldots\ldots (16)$$

Next, transpose of the Cholesky tensor is encoded on the first quantum circuit to form a second Cholesky circuit component as illustrated by block 408. It is mathematically represented by equation 17.

$$V'_L = \sum_{c=0,r_1=0,r_2=0}^{Naux-1,Nao-1} \left[ |c, r_1, r_2, 0\rangle\langle c, r_1, r_2, 0| \otimes \left( L_{r_1,r_2}^{c'} I + i\sqrt{1 - \left(L_{r_1,r_2}^{c'}\right)^2} Y \right) + |c, r_1, r_2, 1\rangle\langle c, r, 1| \otimes I_2 \right] \ldots (17)$$

Further, the second quantum circuit component is composed with the second Cholesky circuit component (to form block 410) for processing the intermediate state vector to obtain a plurality of states at the second set of qubits in the first quantum circuit. At step 210a6, sequences of bitstrings are read from the second set of qubits (by block 412) to obtain the direct matrix. This step is mathematically represented by equation 18.

$$\langle 0, i, 0,0,0 | H^{\otimes m} V'_L R V_L O(D) | 0, j, 0,1,0 \rangle = \sum L_{ij}^a L_{kl}^a D_{kl} = J_{ij} \ldots\ldots (18)$$

Once the direct (J) matrix is obtained, at step 210b, a correlation exchange matrix is determined based on the initial density matrix, the collocation matrix, a Gradient of collocation matrix, and a Hessian of the collocation matrix for meta Generalized Gradient Approximation (meta GGA). The correlation exchange matrix is computed using a second quantum circuit. The second quantum circuit comprises (a) control qubits composed of a first set of qubits and a second set of qubits, and (b) a plurality of ancilla qubits. The first set of qubits is associated with the number of points on the numerical grid, the second set of qubits is associated with the number of atomic orbitals, and the plurality of ancilla qubits are used to store numerical entries of the collocation matrix and the initial density matrix. In an embodiment, the second quantum circuit is mathematically represented as: $log\ N_g$ + $2log\ N_{ao}$ + 2 qubits. Here, $log\ N_g$ represents the first set of qubits, $log\ N_{ao}$ represents the second set of qubits, and 2 qubits represent the plurality of ancilla qubits.

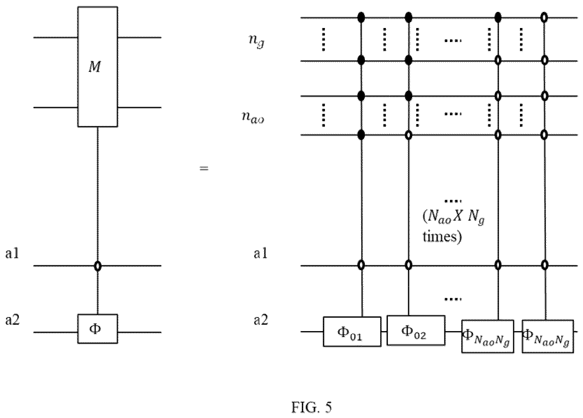

Initially, at step 210b1, the collocation matrix is encoded on (a) control qubits composed of a first set of qubits and a second set of qubits, and (b) a plurality of ancilla qubits in the second quantum circuit to obtain a first quantum circuit block a shown in FIG. 5. FIG. 5 illustrates a first quantum circuit block that encodes a collocation matrix in the method illustrated in FIG. 2, in accordance with some embodiments of the present disclosure. In an embodiment, a quantum circuit block could be an independently functioning quantum circuit or a part of the quantum circuit. In an embodiment, a set of Hadamard gates and a set of multi-controlled RY gates are used to encode the collocation matrix. As shown in FIG. 5, each entry of the collocation matrix is being encoded on $a2$ ancilla qubit with controls on $N_{ao}$ and $N_g$ sets of qubits.

At step 210b2, a gradient of the collocation matrix is computed and encoded on the quantum circuit to obtain a first gradient quantum circuit. The first gradient quantum circuit is obtained by block encoding a plurality of sub-collocation matrices (six sub-collocation matrices in the present disclosure) obtained from the collocation matrix for positions ($x$ +/-delta $x,y,z$), ($x,y$ +/-delta $y, z$), ($x,y,z$ +/-delta $z$) using three additional ancilla qubits. Further, the plurality of sub-collocation matrices are sandwiched across the set of Hadamard gates to block encode finite differences of each of the plurality of sub-collocation matrices for three directions including a X-direction, a Y-direction, and a Z-direction.

At step 210b3, a hessian of the collocation matrix on the quantum circuit is computed and encoded to obtain a first hessian quantum circuit block. The hessian of the collocation matrix is obtained by encoding the collocation matrix for a set of real space grid points (+/-delta, 0,0), (0, +/-delta, 0), (0,0, +/-delta), (+/ delta, 0, +/-delta), (+/delta, +/-delta, 0), (0, +/-delta, +/-delta) on (a) control qubits composed of the first set of qubits and the second set of qubits, and (b) the plurality of ancilla qubits to obtain the third quantum circuit block. In other words, a plurality of collocation matrices are obtained from the collocation matrix with shifted coordinates corresponding to 15 basis functions which are mathematically represented by equation 19

$$\Phi_{\pm\Delta x,\pm\Delta y} = \begin{pmatrix} \phi_1(x_1 \pm \Delta x, y_1 \pm \Delta y, z_1), \ldots\ldots\ldots \phi_1\left(x_{N_g} \pm \Delta x, y_{N_g} \pm \Delta y, z_{N_g}\right) \\ \vdots \\ \phi_{N_{ao}}(x_1 \pm \Delta x, y_1 \pm \Delta y, z_1), \ldots\ldots\ldots \phi_{N_{ao}}\left(x_{N_g} \pm \Delta x, y_{N_g} \pm \Delta y, z_{N_g}\right) \end{pmatrix}$$

$$\Phi_{\pm\Delta x,\pm\Delta z} = \begin{pmatrix} \phi_1(x_1 \pm \Delta x, y_1 y, z_1 \pm \Delta z), \ldots\ldots\ldots \phi_{N_{ao}}\left(x_{N_g} \pm \Delta x, y_{N_g}, z_{N_g} \pm \Delta z\right) \\ \vdots \\ \phi_1(x_1 \pm \Delta x, y_1, z_1 \pm \Delta z), \ldots\ldots\ldots \phi_{N_{ao}}\left(x_{N_g} \pm \Delta x, y_{N_g}, z_{N_g} \pm \Delta z\right) \end{pmatrix}$$

$$\Phi_{\pm\Delta y,\pm\Delta z} = \begin{pmatrix} \phi_1(x_1, y_1 \pm \Delta y, z_1 \pm \Delta z), \ldots\ldots\ldots \phi_{N_{ao}}\left(x_{N_g}, y_{N_g} \pm \Delta y, z_{N_g} \pm \Delta z\right) \\ \vdots \\ \phi_1(x_1, y_1 \pm \Delta y, z_1 \pm \Delta z), \ldots\ldots\ldots \phi_{N_{ao}}\left(x_{N_g}, y_{N_g} \pm \Delta x, z_{N_g} \pm \Delta z\right) \end{pmatrix}$$

$$\Phi_{\pm\Delta x,\pm\Delta y} = \begin{pmatrix} \phi_1(x_1 \pm \Delta x, y_1 \pm \Delta y, z_1), \ldots\ldots\ldots \phi_1\left(x_{N_g} \pm \Delta x, y_{N_g} \pm \Delta y, z_{N_g}\right) \\ \vdots \\ \phi_{N_{ao}}(x_1 \pm \Delta x, y_1 \pm \Delta y, z_1), \ldots\ldots\ldots \phi_{N_{ao}}\left(x_{N_g} \pm \Delta x, y_{N_g} \pm \Delta y, z_{N_g}\right) \end{pmatrix}$$

$$\Phi_{\pm 2\Delta x} = \begin{pmatrix} \phi_1(x_1 \pm 2\Delta x, y_1 y, z_1), \ldots\ldots\ldots \phi_{N_{ao}}\left(x_{N_g} \pm 2\Delta x, y_{N_g}, z_{N_g}\right) \\ \vdots \\ \phi_1(x_1 \pm 2\Delta x, y_1), \ldots\ldots\ldots \phi_{N_{ao}}\left(x_{N_g} \pm 2\Delta x, y_{N_g}, z_{N_g}\right) \end{pmatrix}$$

$$\Phi_{\pm 2\Delta y} = \begin{pmatrix} \phi_1(x_1, y_1 \pm 2\Delta y, z_1 \pm \Delta z), \ldots\ldots\ldots \phi_{N_{ao}}\left(x_{N_g}, y_{N_g} \pm 2\Delta y, z_{N_g}\right) \\ \vdots \\ \phi_1(x_1, y_1 \pm 2\Delta y, z_1 \pm \Delta z), \ldots\ldots\ldots \phi_{N_{ao}}\left(x_{N_g}, y_{N_g} \pm 2\Delta y, z_{N_g}\right) \end{pmatrix}$$

$$\Phi_{\pm 2\Delta z} = \begin{pmatrix} \phi_1(x_1, y_1, z_1 \pm 2\Delta z), \ldots\ldots\ldots \phi_{N_{ao}}\left(x_{N_g}, y_{N_g}, z_{N_g}\right) \\ \vdots \\ \phi_1(x_1, y_1 \pm 2\Delta y, z_1 \pm \Delta z), \ldots\ldots\ldots \phi_{N_{ao}}\left(x_{N_g}, y_{N_g} \pm 2\Delta y, z_{N_g}\right) \end{pmatrix}$$

$$(19)$$

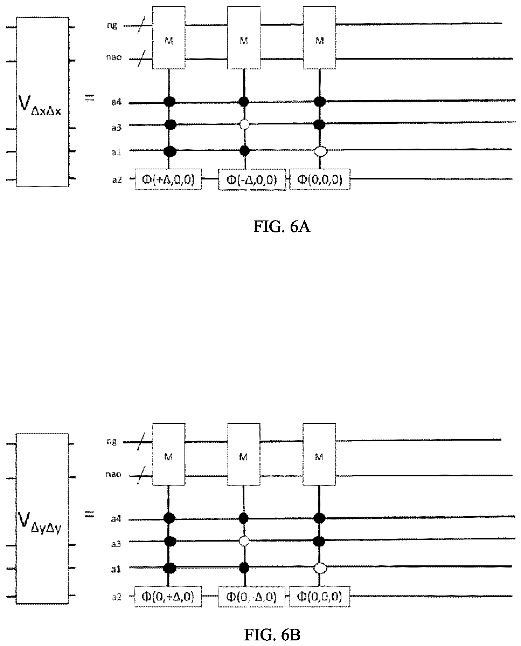

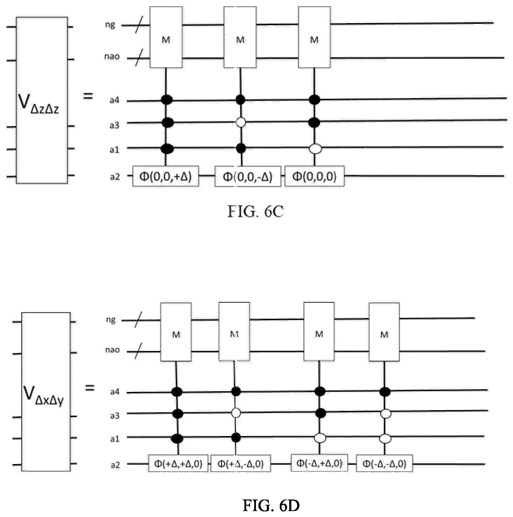

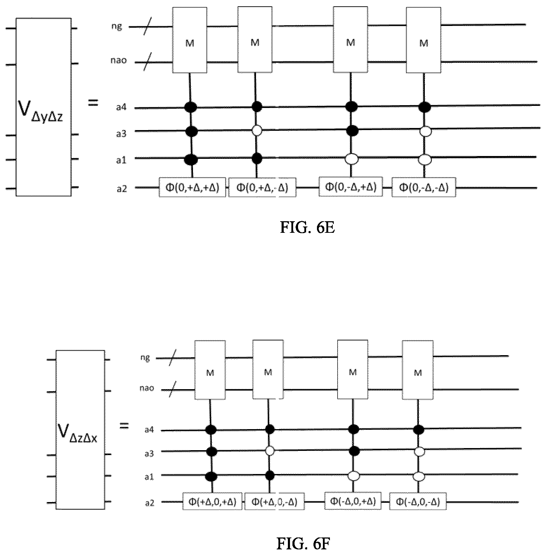

The plurality of collocation matrices for different position shift are: $(x,y,z)$, $(x + /\text{-}delta\ x, y, z)$, $(x, y +/\text{-}delta\ y, z)$, $(x, y, z +/\text{-}delta\ z)$, $(x +/\text{-}delta\ x, y +/\ \text{-}delta\ y, z)$, $(x +/\text{-}delta\ x, y, z +/delta\ z)$, $(x, y +/\text{-}delta\ y, z +/\text{-}delta\ z)$. Altogether 25 collocation matrices are block encoded with 5 qubits. And then, finite differences are performed to compute different components of the Hessian of the collocation matrix as shown in FIGS. 6A through 6F. The finite difference formulas for different components of the Hessian of the collocation matrix is given by,

$$\partial_j^2 \Phi = \Phi_{2\Delta j} + \Phi_{-2\Delta j} - 2\Phi$$

$$\partial_{xy}^2 \Phi = \left(\Phi_{\Delta x,\Delta y} - \Phi_{\Delta x,-\Delta y}\right) - \left(\Phi_{-\Delta x,\Delta y} - \Phi_{-\Delta x,-\Delta y}\right)$$

$$\partial_{yz}^2 \Phi = \left(\Phi_{\Delta y,\Delta z} - \Phi_{\Delta y,-\Delta z}\right) - \left(\Phi_{-\Delta y,\Delta z} - \Phi_{-\Delta y,-\Delta z}\right)$$

$$\partial_{zx}^2 \Phi = \left(\Phi_{\Delta z,\Delta x} - \Phi_{\Delta z,-\Delta x}\right) - \left(\Phi_{-\Delta z,\Delta x} - \Phi_{-\Delta z,-\Delta x}\right) \qquad (20)$$

As shown in equation (20), six independent components of the Hessian of the collocation matrix are obtained. The above 19 matrices are encoded on a third quantum circuit and their differences corresponding to the finite difference formulas are computed. As a first step, the third quantum circuit mathematically expressed as $ng + nao + 2 + 2$ qubits is used, where $n_g = \log N_g$, $n_{ao} = \log N_{ao}$ arranged as $|\rangle N_{ao} |\rangle N_g |\rangle 1 |\rangle a1 |\rangle a2$ to compute gradients of the density matrix as shown in equation (21).

$$
V_{\partial_j^2 \Phi} = I_2^{\otimes(nao+ng)} H^{\otimes 2} I_2^{\otimes 2} H \sum_{r,c} (|c,r,0,0,0\rangle\langle c,r,0,0,0| \otimes \left[ \Phi_{+\Delta j,c,r} I \right.
$$

$$
\left. + i\sqrt{1 - \Phi_{+\Delta j,c,r}^2} Y \right]
$$

$$
+ |c,r,0,0,1\rangle\langle c,r,0,0,1| \otimes I_2 + |c,r,0,1,0\rangle\langle c,r,0,1,0| \otimes \left[ \Phi_{-\Delta j,c,r} I \right.
$$

$$
\left. + i\sqrt{1 - \Phi_{-\Delta j,c,r}^2} Y \right] + |c,r,0,1,1\rangle\langle c,r,0,1,1| \otimes I_2
$$

$$
+ |c,r,1,0,0\rangle\langle c,r,1,0,0| \otimes \left[ \Phi_{c,r} I + i\sqrt{1 - \Phi_{c,r}^2} Y \right]
$$

$$
+ |c,r,1,0,1\rangle\langle c,r,1,0,1| \otimes I_2
$$

$$
+ |c,r,1,1\rangle\langle c,r,1,1| \otimes I_2^{\otimes 2}) I_2^{\otimes(nao+ng)} H^{\otimes 2} I_2^{\otimes 2} H
$$

$$
V_{\partial_{ij}^2 \Phi} = I_2^{\otimes(nao+ng)} H^{\otimes 2} I_2^{\otimes 2} H \sum_{r,c} (|c,r,0,0,0\rangle\langle c,r,0,0,0| \otimes \left[ \Phi_{+\Delta i,+\Delta j,c,r} I \right.
$$

$$
\left. + i\sqrt{1 - \Phi_{+\Delta i,+\Delta j,c,r}^2} Y \right]
$$

$$
+ |c,r,0,0,1\rangle\langle c,r,0,0,1| \otimes I_2 + |c,r,0,1,0\rangle\langle c,r,0,1,0|
$$

$$
\otimes \left[ \Phi_{-\Delta i,+\Delta j,c,r} I + i\sqrt{1 - \Phi_{-\Delta i,+\Delta j,c,r}^2} Y \right]
$$

$$
+ |c,r,0,1,1\rangle\langle c,r,0,1,1| \otimes I_2
$$

$$
+ |c,r,1,0,0\rangle\langle c,r,1,0,0| \otimes \left[ \Phi_{+\Delta i,-\Delta j,c,r} I + i\sqrt{1 - \Phi_{+\Delta i,-\Delta j,c,r}^2} Y \right]
$$

$$
+ |c,r,1,0,1\rangle\langle c,r,1,0,1| \otimes I_2
$$

$$
+ |c,r,1,1,0\rangle\langle c,r,1,1,0| \otimes \left[ \Phi_{-\Delta i,-\Delta j,c,r} I + i\sqrt{1 - \Phi_{-\Delta i,-\Delta j,c,r}^2} Y \right]
$$

$$
+ |c,r,1,1,1\rangle\langle c,r,1,1,1| \otimes I_2) I_2^{\otimes(nao+ng)} H^{\otimes 2} I_2^{\otimes 2} H
$$

$$
(21)
$$

At step 210b4, the first quantum circuit block is composed with a second quantum circuit block to construct a third quantum circuit block. The second quantum circuit block encodes the initial density matrix on (a) control qubits composed of the second set of qubits and (b) an ancilla qubit in the quantum circuit. The initial density matrix is loaded using its Eigen decomposition representation that involves: i) $^{Nao}C_2$ Givens rotations on $\log N_{ao}$ qubits, ii) Then block encoding a diagonal matrix of 1's in the initial $N_e/2$ diagonal blocks and zeros in the rest using $2\log N_{ao}$ qubits, iii) Another set of $^{Nao}C_2$ Givens rotation with conjugate angles on $N_e/2$ diagonals. This is mathematically represented by equation 13. The third quantum circuit block encodes a matrix multiplication of the collocation matrix and the initial density matrix.

At step 210b5, an electronic density is encoded on the quantum circuit by performing a sequential matrix multiplication of the collocation matrix, the density matrix, and the collocation matrix to obtain a fourth quantum circuit block. After obtaining (i) the first quantum circuit block for encoding the collocation matrix, and (ii) the second quantum circuit block for encoding the initial density matrix, another collocation matrix is block-encoded for matrix multiplication using the fourth quantum circuit block. The matrix multiplication encodes the data for electronic density on log Ng qubits. This is a state preparation oracle for loading the electronic density.

At step 210b6, the third quantum circuit block that encodes the matrix multiplication of the collocation matrix and the initial density matrix is composed with the first gradient quantum circuit block that encoded the gradient of the collocation matrix, to construct a second gradient quantum circuit block that encodes a gradient of the electronic density of the chemical compound. This is obtained when another set of three gradient matrices which are obtained by computing the gradient of the collocation matrix are block encoded using two ancilla qubits. The matrix multiplication in the second gradient quantum circuit block encodes the data for the gradient of electronic density on $log N_g + 2$ qubits and the gradient of the collocation. This is the state preparation oracle for loading the gradient of the electronic density.

At step 210b7, the first hessian quantum circuit block that encodes the hessian of the collocation matrix is composed sequentially with the first quantum circuit block and the second quantum circuit block that encodes the initial density matrix to construct a second hessian quantum circuit block that encodes a first component of hessian of the electronic density. The first component of the hessian of the electronic density is obtained by a sequential matrix multiplication of the hessian of the collocation matrix, the collocation matrix, and the initial density matrix. Similarly, at steps 210b8, the first hessian quantum circuit block that encodes the hessian of the collocation matrix is composed sequentially with the second quantum circuit block that encodes the initial density matrix and the first quantum circuit block that encoded the collocation matrix to construct a third hessian quantum circuit block that encodes a second component of hessian of the electronic density. The second component of the hessian of the electronic density is obtained by a sequential matrix multiplication of the hessian of the collocation matrix, the initial density matrix, and the collocation matrix.

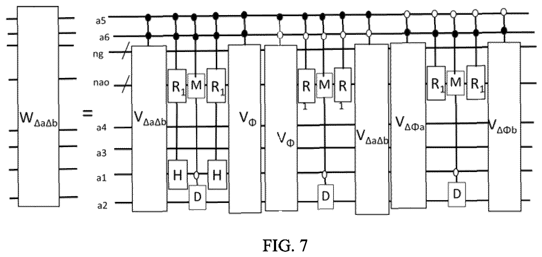

Further, at step 210b9, a fourth hessian quantum circuit block is constructed that encodes a third component of hessian of the electronic density of the chemical compound by sequentially composing the first hessian quantum circuit block that encodes the hessian of the collocation matrix with the second quantum circuit block that encodes the initial density matrix and the first gradient quantum circuit block encodes the gradient of the collocation matrix. The third component of the hessian of the electronic density is obtained by a sequential matrix multiplication of the hessian of the collocation matrix, the initial density matrix, and the gradient of the collocation matrix. FIG. 7 illustrates a quantum circuit comprising the second hessian quantum circuit block, the third hessian quantum circuit block, and the fourth hessian quantum circuit block that encode the first, second and third components of the hessian of electronic density respectively in the method illustrated in FIG. 2, in accordance with some embodiments of the present disclosure.

Further, at step 210b10, the hessian of the electronic density of the chemical compound is computed by adding the first, second and third component of hessian of the electronic density of the chemical compound using the plurality of ancilla qubits. This step is mathematically represented by equation (22).

$$\partial_{ij}^2 \left( \rho(r_1), \dots \rho\left(r_{N_g}\right) \right)^T = \partial_i \Phi^T D \partial_j \Phi + \partial_{ij}^2 \Phi^T D \Phi + \Phi^T D \partial_{ij}^2 \Phi \quad (22)$$

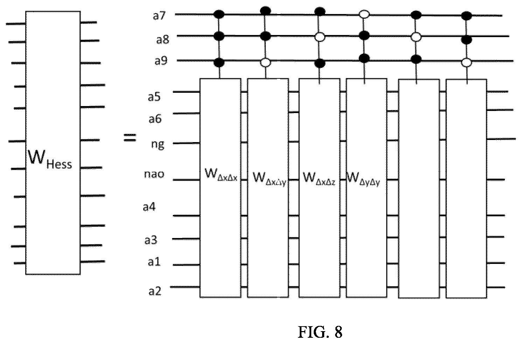

Furthermore, at step 210b11, the Hessian of the electronic density of the chemical compound is encoded on (a) control qubits composed of a first set of qubits and a second set of qubits, and (b) the plurality of ancilla qubits to obtain a fifth hessian quantum circuit block. FIG. 8 illustrates a fifth quantum circuit block that encodes Hessian of the electronic density in the method illustrated in FIG. 2, in accordance with some embodiments of the present disclosure. The fifth hessian quantum circuit block is mathematically represented by equation (23).

$$\partial_{ij}^2 \rho(x, y, z) = \langle 0,0,0,0,c,0,0| H^{\otimes 2}(|0,0\rangle\langle 0,0| \otimes V_{\partial i\Phi} R_1 V_D V_{\partial_j\Phi} +$$

$$|0,1\rangle\langle 0,1| \otimes V_{\partial_{ij}^2\Phi} R_1 V_D V_\Phi + |1,0\rangle\langle 1,0| \otimes V_\Phi R_1 V_D V_{\partial_{ij}^2\Phi} +$$

$$|11\rangle\langle 11| \otimes I_2^{\otimes(ng+nao+2+2)} ) H^{\otimes 2} |0,0,0,0,c,0,0\rangle \quad (23)$$

At step 210b12, the fourth quantum circuit block is put with a fifth quantum circuit block for a phase angle shifted reflector, the second gradient quantum circuit block that encodes the gradient of the electronic density, and the fifth hessian quantum circuit block that encodes the hessian of the electronic density into a quantum signal processing sequence. The quantum signal processing sequence create a sixth quantum circuit block that encodes a Chebyshev polynomial approximation of a nonlinear function of the electronic density. The nonlinear function is a derivative of the electronic energy density with respect to the electronic density. This means a quantum signal processing sequence is constructed to encode a meta-GGA density functional.

At step 210b13, the sixth quantum circuit block is composed with the first quantum circuit block, to create a seventh quantum circuit block that encodes a $Z$ matrix. This step is mathematically represented by equation (24)

$$V_Z = \prod_{k=1}^{N} [(H^{\otimes nao} \otimes I^{\otimes(ng+2)}) V_\Phi R_1 V_D R_1 V_\Phi ((1 + e^{-i\phi_k})|0, i, 0, 0\rangle\langle 0, i, 0, 0| - 1)]$$

$$\prod_{k=1}^{N} [(H^{\otimes nao} \otimes I^{\otimes(ng+2)}) V_{\nabla\Phi} R_1 V_D R_1 V_\Phi ((1 + e^{-i\phi_k})|0, i, 0, 0\rangle\langle 0, i, 0, 0| - 1)]$$

$$\prod_{k=1}^{N} [(H^{\otimes nao} \otimes I^{\otimes(ng+2)}) V_{\nabla\Phi} R_1 V_D R_1 V_{\nabla\Phi} ((1 + e^{-i\phi_k})|0, i, 0, 0\rangle\langle 0, i, 0, 0| - 1)]$$

$$\prod_{k=1}^{N} [(H^{\otimes nao} \otimes I^{\otimes(ng+2)}) V_{\nabla^2\Phi} R_1 V_D R_1 V_\Phi ((1 + e^{-i\phi_k})|0, i, 0, 0\rangle\langle 0, i, 0, 0| - 1)]$$

$$\prod_{k=1}^{N} [(H^{\otimes nao} \otimes I^{\otimes(ng+2)}) V_\Phi R_1 V_D R_1 V_{\nabla^2\Phi} ((1 + e^{-i\phi_k})|0, i, 0, 0\rangle\langle 0, i, 0, 0| - 1)]$$

$$(H^{\otimes nao} \otimes I^{\otimes(ng+2)}) V_\Phi H^{\otimes ng} \qquad (24)$$

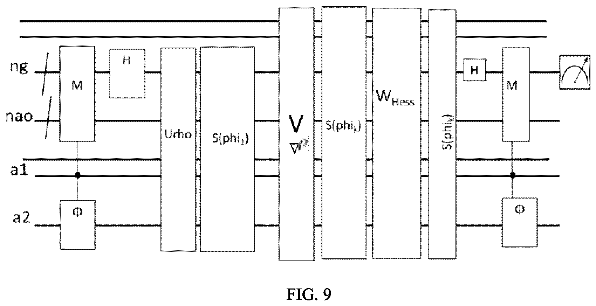

Further, at step 210b14, the seventh quantum circuit block is composed with the first quantum circuit block, to create an eight quantum circuit block that encodes the correlation exchange matrix. FIG. 9 illustrates the eight quantum circuit block that encodes the correlation exchange matrix in the method illustrated in FIG. 2, in accordance with some embodiments of the present disclosure. As shown in FIG. 9, In order to compute the Hessian contribution to the exchange potential, a gradient of energy density needs to be computed with respect to the electronic density. The energy density is a nonlinear function of the electronic density, its gradient and its Hessian. A quantum signal processing sequence is generated using the block encoding oracles of the electronic density, its 3 components of the gradient and 6 components of the Hessian to obtain the electronic energy density and its derivative with respect to its density. This is then sandwiched via the first quantum circuit block that encodes the Collocation matrix to create the correlation exchange matrix, $V^{xc}$. This step is mathematically represented by equation (25).

$$V_{ij}^{xc} = \langle i, 0, 0, 0 | H^{\otimes ng} V_\Phi V_Z | j, 0, 0, 0 \rangle = \sum_r \Phi_{ir} \frac{dE(\rho, \nabla\rho, \nabla^2\rho)}{d\rho(r)} \Phi_{jr} \ (25)$$

Once the correlation exchange matrix is determined, at step 210c, a Fock matrix is computed by adding the direct matrix and the correlation exchange matrix. At step 210d, qubitized diagonalization of the Fock matrix is performed to obtain an intermediate density matrix. At step 210e, the steps 210a through 210d are repeated until the convergence criteria is satisfied by considering the intermediate density matrix as the initial density matrix to obtain the final density matrix of the chemical compound.

Finally, at step 212, the one or more classical hardware processors are configured to utilize the final density matrix of the chemical compound to extract the one or more properties of the chemical compound. The final density matrix enables computation of properties such as the HOMO-LUMO gap from energies of the highest occupied Kohn Sham orbital and lowest unoccupied orbital, the charge distribution from integrating the electronic density in regions around the different atoms where the electronic density is obtained from the initial density matrix, a dipole moment from an expectation value of perturbation electric field operator with respect to the final density matrix.

USE CASE EXAMPLES

Example 1: For drug screening, an accurate potential energy surface of a drug molecule is obtained from the method 200 disclosed herein. Then, gradients are studied from the PES and the forces associated with the displacement of the ligand molecules in protein environment are calculated. This will enable more accurate assessment of ligand conformations and protein docking faces. Further dynamical response functions can be calculated which helps better determination of the binding.

Example 2: For selecting cathode materials for rechargeable batteries, accurate optimized geometry of the cathode molecules in electrolyte environment, and associated ground state energy are calculated from method 200. This will then be used to compute more accurate energy enthalpy differences for the redox reactions. In turn the energy enthalpy differences are used to calculate high open cell voltages(OCV) (equilibrium voltage). A higher OCV leads to a higher cut-off for the recharging voltage. Cathode materials with different co-doped transition metal oxides (like Co, Ni, Mn) are screened using high OCV as the deciding factor. As there are large number of properties to optimize such as number of battery recharging cycles, charging percentage, time to recharge, weight of battery material. The number of battery materials are more than a million, thus faster quantum chemistry calculation is required to screen them based on Density functional theory.

Example 3: For drug conformational search and drug design, the molecular descriptors, conformations, solvation energy is obtained from the method 200 disclosed herein. Then from the conformational energies and the descriptors the subset of drug molecule structure and conformations with one or more target properties are prepared via high throughput experimentation.

Example 4: Screening corrosion inhibitor molecules for aluminum sheets where efficacy is required to be enhanced, low cost of inhibitor molecules and availability is maintained, and adverse medical effects are ensured.

EXPERIMENTAL RESULTS

The meta generalized gradient approximation (meta GGA) functional computation was performed on classical processor using conventional methods and quantum processor using the method 200. The classical complexity is measured in terms of space and time complexity. Similarly, the quantum complexity is measured in terms of number of qubits and gate complexity. The results are recorded in table 1.

Table 1

| Quantity | Classical Complexity | | Quantum Complexity | |
|---|---|---|---|---|
| | Space | Time | No.of Qubits | Gate complexity |
| $V_{xc}[\rho, \nabla\rho, \nabla^2\rho]$(meta - GGA) | $O(N^2 N_g)$ | $O(2N^2 N_g)$ | $\log N + \log N_g$ | $16(\log N + \log N_g)$ |

For the meta GGA density functional computation, the correlation exchange potential is a function of the electronic density, gradient of the electronic density and the Hessian of the electronic density. For computing the electronic density, the number of qubits needed to encode the electronic density is given by $\log N + \log N_g$, where $N_g$ is number of points on numerical grid and $N$ is number of basis functions. Further, 3 additional ancilla qubits are needed to encode different components of the Hessian of the electronic density. Gate complexity is associated with a diffusion operator that enables a multiplication between the Collocation matrix and the Hessian of the collocation matrix and the initial density matrix. The gate complexity scales logarithmically with the number of basis functions. Classically, the space complexity to store the integrals is $N^2 N_g$ and the time complexity is $N^2 N_g$.

The written description describes the subject matter herein to enable any person skilled in the art to make and use the embodiments. The scope of the subject matter embodiments is defined by the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, non-volatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A method (200), performed by a system comprising one or more classical hardware processors and a plurality of unentangled Quantum Processor Units, QPUs, wherein the one or more classical hardware processors are communicably coupled to the plurality of unentangled QPUs by respective interfaces, wherein the method comprises:

   receiving, via the one or more classical hardware processors, a chemical compound whose one or more properties are to be extracted, wherein the chemical compound is at least one of (i) a molecule, and (ii) a solid (202);
   obtaining, via the one or more classical hardware processors, a plurality of atomic coordinates of each of a plurality of atoms present in the chemical compound (204);
   determining, via the one or more classical hardware processors, (i) a plurality of electron integrals, (ii) a core Hamiltonian matrix, and (iii) a collocation matrix, from the plurality of atomic coordinates of each of the plurality of atoms present in the chemical compound, wherein the collocation matrix is a rectangular matrix of dimension $N_g$ and $N_{ao}$, and wherein $N_g$ represents a number of real space grid points, $N_{ao}$ is a number of basis functions (206);
   determining, via the plurality of unentangled QPUs, an initial density matrix of the chemical compound from the core Hamiltonian matrix, wherein the initial density matrix is constructed using (i) a single particles unitary rotation matrix which is obtained by diagonalizing the core Hamiltonian matrix, and (ii) electron occupancies (208);
   iteratively updating, via the plurality of unentangled QPUs, the initial density matrix until a convergence criteria is satisfied to obtain a final density matrix of the chemical compound (210), wherein iteratively updating the initial density matrix at each iteration comprises:

      (a) computing a direct, J, matrix from the initial density matrix (210a);
      (b) determining a correlation exchange matrix based on the initial density matrix, the collocation matrix, a Gradient of collocation matrix, and a Hessian of the collocation matrix for meta Generalized Gradient Approximation, meta GGA, (210b) by:

         (i) encoding the collocation matrix on (a) control qubits composed of a first set of qubits and a second set of qubits, and (b) a plurality of ancilla qubits in a second quantum circuit, to obtain a first quantum circuit block, wherein the first set of qubits is associated with the number of points on the numerical grid, the second set of qubits is associated with the number of atomic orbitals, and the plurality of ancilla qubits are used to store numerical entries of the collocation matrix and the initial density matrix (210b1);
         (ii) encoding a gradient of the collocation matrix on the quantum circuit to obtain a gradient to obtain a first gradient quantum circuit block;
         (iii) encoding a hessian of the collocation matrix on the quantum circuit to obtain a first hessian quantum circuit block (210b2);

(iv) composing the first quantum circuit block with a second quantum circuit block that encodes an intermediate density matrix on (a) control qubits composed of the second set of qubits and (b) an ancilla qubit in the quantum circuit, to construct a third quantum circuit block (210b3);

(v) encoding an electronic density on the quantum circuit by performing a sequential matrix multiplication of the collocation matrix, the density matrix, and the collocation matrix to obtain a fourth quantum circuit block (210b4);

(vi) composing the third quantum circuit block with the first gradient quantum circuit block that encoded the gradient of the collocation matrix, to construct a second gradient quantum circuit block that encodes a gradient of the electronic density of the chemical compound (210b5);

(vii) composing the first hessian quantum circuit block that encodes the hessian of the collocation matrix with the first quantum circuit block and the second quantum circuit block that encodes the initial density matrix sequentially, to construct a second hessian quantum circuit block that encodes a first component of hessian of the electronic density (210b6);

(viii) composing the first hessian quantum circuit block that encodes the hessian of the collocation matrix with the second quantum circuit block that encodes the initial density matrix and the first quantum circuit block that encoded the collocation matrix sequentially, to construct a third hessian quantum circuit block that encodes a second component of hessian of the electronic density (210b7);

(ix) composing the first hessian quantum circuit block that encodes the hessian of the collocation matrix with the second quantum circuit block that encodes the initial density matrix and the first gradient quantum circuit block encodes the gradient of the collocation matrix, to construct a fourth hessian quantum circuit block that encodes a third component of hessian of the electronic density of the chemical compound (210b8);

(x) computing the hessian of the electronic density of the chemical compound by adding the first, second and third component of hessian of the electronic density of the chemical compound using the plurality of ancilla qubits (210b9);

(xi) encoding the Hessian of the electronic density of the chemical compound on (a) control qubits composed of a first set of qubits and a second set of qubits, and (b) the plurality of ancilla qubits, to obtain a fifth hessian quantum circuit block (210b10);

(xii) putting the fourth quantum circuit block with a fifth quantum circuit block for a phase angle shifted reflector, the second gradient quantum circuit block that encodes the gradient of the electronic density, and the fifth hessian quantum circuit block that encodes the hessian of the electronic density into a quantum signal processing sequence, to create a sixth quantum circuit block that encodes a Chebyshev polynomial approximation of a nonlinear function of the electronic density, wherein the nonlinear function is a derivative of the electronic energy density with respect to the electronic density (210b11);

(xiii) composing the sixth quantum circuit block with the first quantum circuit block, to create a seventh quantum circuit block that encodes a $Z$ matrix (210b12); and

(xiv) composing the seventh quantum circuit block with the first quantum circuit block, to create an eight quantum circuit block that encodes the correlation exchange matrix (210b13 );

(c) determining a Fock, F, matrix by adding the $J$ matrix and the correlation exchange matrix (210c);

(d) performing a qubitized diagonalization of the $F$ matrix to obtain an intermediate density matrix (210d); and

(e) repeating the steps (a) through (d) until the convergence criteria is satisfied to obtain the final density matrix of the chemical compound, wherein the intermediate density matrix is considered as the initial density matrix until the convergence criteria is satisfied (210e); and

utilizing, via the one or more classical hardware processors, the final density matrix of the chemical compound to extract the one or more properties of the chemical compound (212).

2. The method as claimed in claim 1, wherein the convergence criteria is satisfied when a norm of a difference between the intermediate density matrix at a current iteration and the initial density matrix at a previous iteration is lesser than a predefined threshold.

3. The method as claimed in claim 1, wherein the (i) control qubits composed of the first set of qubits and the second set of qubits, and (ii) the plurality of ancilla qubits, to obtain the first quantum circuit block, is mathematically represented as: $log\,N_g + log\,N_{ao} + 2\,qubits,$ where $log\,N_g$ represents the first set of qubits, $log\,N_{ao}$ represents the second set of qubits, and 2 qubits represent the plurality of ancilla qubits.

4.  The method as claimed in claim 1, wherein the hessian of the collocation matrix is obtained by encoding the collocation matrix for a set of real space grid points $(+/-delta, 0,0)$, $(0, +/-delta, 0)$, $(0,0, +/-delta)$, $(+/delta, 0, +/-delta)$, $(+/delta, +/-delta, 0)$, $(0, +/-delta, +/-delta)$ on (a) control qubits composed of the first set of qubits and the second set of qubits, and (b) the plurality of ancilla qubits to obtain the first hessian quantum circuit block.

5.  A system (100), comprising:

    one or more classical hardware processors (108) and a plurality of unentangled quantum processors (122), wherein the one or more classical hardware processors (108) are communicably coupled to the plurality of unentangled quantum processors (122) by respective interfaces, wherein the one or more classical hardware processors are communicably coupled to at least one memory (110) storing programmed instructions; one or more Input /Output (I/O) interfaces (116); and the plurality of unentangled quantum processors (122) are operatively coupled to the at least one quantum memory (124), wherein the one or more classical hardware processors (108) and the plurality of unentangled quantum processors (122) are configured by the programmed instructions to:

    receive, via the one or more classical hardware processors, a chemical compound whose one or more properties are to be extracted, wherein the chemical compound is at least one of (i) a molecule, and (ii) a solid;

    obtain, via the one or more classical hardware processors, a plurality of atomic coordinates of each of a plurality of atoms present in the chemical compound;

    determine, via the one or more classical hardware processors, (i) a plurality of electron integrals, (ii) a core Hamiltonian matrix, and (iii) a collocation matrix, from the plurality of atomic coordinates of each of the plurality of atoms present in the chemical compound, wherein the collocation matrix is a rectangular matrix of dimension $N_g$ and $N_{ao}$, and wherein $N_g$ represents a number of real space grid points, $N_{ao}$ is a number of basis functions;

    determine, via the plurality of unentangled quantum processors, an initial density matrix of the chemical compound from the core Hamiltonian matrix, wherein the initial density matrix is constructed using (i) a single particles unitary rotation matrix which is obtained by diagonalizing the core Hamiltonian matrix, and (ii) electron occupancies;

    iteratively update, via the plurality of unentangled quantum processors, the initial density matrix until a convergence criteria is satisfied to obtain a final density matrix of the chemical compound, wherein iteratively updating the initial density matrix at each iteration comprises:

    (a) computing a direct, J, matrix from the initial density matrix;
    (b) determining a correlation exchange matrix based on the initial density matrix, the collocation matrix, a Gradient of collocation matrix, and a Hessian of the collocation matrix for meta Generalized Gradient Approximation, meta GGA, by:

    (i) encoding the collocation matrix on (a) control qubits composed of a first set of qubits and a second set of qubits, and (b) a plurality of ancilla qubits in a second quantum circuit, to obtain a first quantum circuit block, wherein the first set of qubits is associated with the number of points on the numerical grid, the second set of qubits is associated with the number of atomic orbitals, and the plurality of ancilla qubits are used to store numerical entries of the collocation matrix and the initial density matrix;
    (ii) encoding a gradient of the collocation matrix on the quantum circuit to obtain a gradient to obtain a first gradient quantum circuit block;
    (iii) encoding a hessian of the collocation matrix on the quantum circuit to obtain a first hessian quantum circuit block;
    (iv) composing the first quantum circuit block with a second quantum circuit block that encodes an intermediate density matrix on (a) control qubits composed of the second set of qubits and (b) an ancilla qubit in the quantum circuit, to construct a third quantum circuit block;
    (v) encoding an electronic density on the quantum circuit by performing a sequential matrix multiplication of the collocation matrix, the density matrix, and the collocation matrix to obtain a fourth quantum circuit block;
    (vi) composing the third quantum circuit block with the first gradient quantum circuit block that encoded the gradient of the collocation matrix, to construct a second gradient quantum circuit block that encodes a gradient of the electronic density of the chemical compound;
    (vii) composing the first hessian quantum circuit block that encodes the hessian of the collocation matrix with the first quantum circuit block and the second quantum circuit block that encodes the initial density matrix sequentially, to construct a second hessian quantum circuit block that encodes a first component of hessian of the electronic density;
    (viii) composing the first hessian quantum circuit block that encodes the hessian of the collocation matrix with the second quantum circuit block that encodes the initial density matrix and the first quantum circuit block that encoded the collocation matrix sequentially, to construct a third hessian quantum circuit block that encodes a second component of hessian of the electronic density;

(ix) composing the first hessian quantum circuit block that encodes the hessian of the collocation matrix with the second quantum circuit block that encodes the initial density matrix and the first gradient quantum circuit block encodes the gradient of the collocation matrix, to construct a fourth hessian quantum circuit block that encodes a third component of hessian of the electronic density of the chemical compound;

(x) computing the hessian of the electronic density of the chemical compound by adding the first, second and third component of hessian of the electronic density of the chemical compound using the plurality of ancilla qubits;

(xi) encoding the Hessian of the electronic density of the chemical compound on (a) control qubits composed of a first set of qubits and a second set of qubits, and (b) the plurality of ancilla qubits, to obtain a fifth hessian quantum circuit block;

(xii) putting the fourth quantum circuit block with a fifth quantum circuit block for a phase angle shifted reflector, the second gradient quantum circuit block that encodes the gradient of the electronic density, and the fifth hessian quantum circuit block that encodes the hessian of the electronic density into a quantum signal processing sequence, to create a sixth quantum circuit block that encodes a Chebyshev polynomial approximation of a nonlinear function of the electronic density, wherein the nonlinear function is a derivative of the electronic energy density with respect to the electronic density;

(xiii) composing the sixth quantum circuit block with the first quantum circuit block, to create a seventh quantum circuit block that encodes a Z matrix; and

(xiv) composing the seventh quantum circuit block with the first quantum circuit block, to create an eight quantum circuit block that encodes the correlation exchange matrix;

(c) determining a Fock, F, matrix by adding the $J$ matrix and the correlation exchange matrix;

(d) performing a qubitized diagonalization of the $F$ matrix to obtain an intermediate density matrix; and

(e) repeating the steps (a) through (d) until the convergence criteria is satisfied to obtain the final density matrix of the chemical compound, wherein the intermediate density matrix is considered as the initial density matrix until the convergence criteria is satisfied; and

utilize, via the one or more classical hardware processors, the final density matrix of the chemical compound to extract the one or more properties of the chemical compound.

6. The system as claimed in claim 5, wherein the convergence criteria is satisfied when a norm of a difference between the intermediate density matrix at a current iteration and the initial density matrix at a previous iteration is lesser than a predefined threshold.

7. The system as claimed in claim 5, wherein the (i) control qubits composed of the first set of qubits and the second set of qubits, and (ii) the plurality of ancilla qubits, to obtain the first quantum circuit block, is mathematically represented as: $log\ N_g + log\ N_{ao} + 2\ qubits,$ where $log\ N_g$ represents the first set of qubits, $log\ N_{ao}$ represents the second set of qubits, and 2 qubits represent the plurality of ancilla qubits.

8. The system as claimed in claim 5, wherein the hessian of the collocation matrix is obtained by encoding the collocation matrix for a set of real space grid points (+/ -delta, 0,0), (0, +/-delta, 0), (0,0, +/-delta), (+/delta, 0, +/-delta), (+/ delta, +/-delta, 0), (0, +/-delta, +/-delta) on (a) control qubits composed of the first set of qubits and the second set of qubits, and (b) the plurality of ancilla qubits to obtain the first hessian quantum circuit block.

9. One or more non-transitory machine-readable information storage mediums comprising one or more instructions which when executed by one or more classical hardware processors and a plurality of unentangled quantum processors, QPUs, cause:

receiving, via the one or more classical hardware processors, a chemical compound whose one or more properties are to be extracted, wherein the chemical compound is at least one of (i) a molecule, and (ii) a solid;

obtaining, via the one or more classical hardware processors, a plurality of atomic coordinates of each of a plurality of atoms present in the chemical compound ;

determining, via the one or more classical hardware processors, (i) a plurality of electron integrals, (ii) a core Hamiltonian matrix, and (iii) a collocation matrix, from the plurality of atomic coordinates of each of the plurality of atoms present in the chemical compound, wherein the collocation matrix is a rectangular matrix of dimension $N_g$ and $N_{ao}$, and wherein $N_g$ represents a number of real space grid points, $N_{ao}$ is a number of basis functions; determining, via the plurality of unentangled QPUs, an initial density matrix of the chemical compound from the core Hamiltonian matrix, wherein the initial density matrix is constructed using (i) a single particles unitary rotation matrix which is obtained by diagonalizing the core Hamiltonian matrix, and (ii) electron occupancies; iteratively updating, via the plurality of unentangled QPUs, the initial density matrix until a convergence criteria is satisfied to obtain a final density matrix of the chemical compound , wherein iteratively updating the initial density matrix at each iteration comprises:

(a) computing a direct, J, matrix from the initial density matrix;

(b) determining a correlation exchange matrix based on the initial density matrix, the collocation matrix, a Gradient of collocation matrix, and a Hessian of the collocation matrix for meta Generalized Gradient Approximation, meta GGA, by:

(i) encoding the collocation matrix on (a) control qubits composed of a first set of qubits and a second set of qubits, and (b) a plurality of ancilla qubits in a second quantum circuit, to obtain a first quantum circuit block, wherein the first set of qubits is associated with the number of points on the numerical grid, the second set of qubits is associated with the number of atomic orbitals, and the plurality of ancilla qubits are used to store numerical entries of the collocation matrix and the initial density matrix;

(ii) encoding a gradient of the collocation matrix on the quantum circuit to obtain a gradient to obtain a first gradient quantum circuit block;

(iii) encoding a hessian of the collocation matrix on the quantum circuit to obtain a first hessian quantum circuit block;

(iv) composing the first quantum circuit block with a second quantum circuit block that encodes an intermediate density matrix on (a) control qubits composed of the second set of qubits and (b) an ancilla qubit in the quantum circuit, to construct a third quantum circuit block ;

(v) encoding an electronic density on the quantum circuit by performing a sequential matrix multiplication of the collocation matrix, the density matrix, and the collocation matrix to obtain a fourth quantum circuit block;

(vi) composing the third quantum circuit block with the first gradient quantum circuit block that encoded the gradient of the collocation matrix, to construct a second gradient quantum circuit block that encodes a gradient of the electronic density of the chemical compound;

(vii) composing the first hessian quantum circuit block that encodes the hessian of the collocation matrix with the first quantum circuit block and the second quantum circuit block that encodes the initial density matrix sequentially, to construct a second hessian quantum circuit block that encodes a first component of hessian of the electronic density;

(viii) composing the first hessian quantum circuit block that encodes the hessian of the collocation matrix with the second quantum circuit block that encodes the initial density matrix and the first quantum circuit block that encoded the collocation matrix sequentially, to construct a third hessian quantum circuit block that encodes a second component of hessian of the electronic density;

(ix) composing the first hessian quantum circuit block that encodes the hessian of the collocation matrix with the second quantum circuit block that encodes the initial density matrix and the first gradient quantum circuit block encodes the gradient of the collocation matrix, to construct a fourth hessian quantum circuit block that encodes a third component of hessian of the electronic density of the chemical compound;

(x) computing the hessian of the electronic density of the chemical compound by adding the first, second and third component of hessian of the electronic density of the chemical compound using the plurality of ancilla qubits;

(xi) encoding the Hessian of the electronic density of the chemical compound on (a) control qubits composed of a first set of qubits and a second set of qubits, and (b) the plurality of ancilla qubits, to obtain a fifth hessian quantum circuit block;

(xii) putting the fourth quantum circuit block with a fifth quantum circuit block for a phase angle shifted reflector, the second gradient quantum circuit block that encodes the gradient of the electronic density, and the fifth hessian quantum circuit block that encodes the hessian of the electronic density into a quantum signal processing sequence, to create a sixth quantum circuit block that encodes a Chebyshev polynomial approximation of a nonlinear function of the electronic density, wherein the nonlinear function is a derivative of the electronic energy density with respect to the electronic density ;

(xiii) composing the sixth quantum circuit block with the first quantum circuit block, to create a seventh quantum circuit block that encodes a *Z* matrix; and
(xiv) composing the seventh quantum circuit block with the first quantum circuit block, to create an eight quantum circuit block that encodes the correlation exchange matrix ;

(c) determining a Fock, F, matrix by adding the *J* matrix and the correlation exchange matrix;
(d) performing a qubitized diagonalization of the *F* matrix to obtain an intermediate density matrix; and
(e) repeating the steps (a) through (d) until the convergence criteria is satisfied to obtain the final density matrix of the chemical compound, wherein the intermediate density matrix is considered as the initial density matrix until the convergence criteria is satisfied; and

utilizing, via the one or more classical hardware processors, the final density matrix of the chemical compound to extract the one or more properties of the chemical compound.

10. The one or more non-transitory machine-readable information storage mediums as claimed in claim 9, wherein the convergence criteria is satisfied when a norm of a difference between the intermediate density matrix at a current iteration and the initial density matrix at a previous iteration is lesser than a predefined threshold.

11. The one or more non-transitory machine-readable information storage mediums as claimed in claim 9, wherein the (i) control qubits composed of the first set of qubits and the second set of qubits, and (ii) the plurality of ancilla qubits, to obtain the first quantum circuit block, is mathematically represented as: $log\ N_g + log\ N_{ao} + 2$ *qubits,* where $log\ N_g$ represents the first set of qubits, $log\ N_{ao}$ represents the second set of qubits, and 2 qubits represent the plurality of ancilla qubits.

12. The one or more non-transitory machine-readable information storage mediums as claimed in claim 9, wherein the hessian of the collocation matrix is obtained by encoding the collocation matrix for a set of real space grid points (*+/-delta, 0,0*), (0, *+/ -delta,* 0)*, (0,0, +/-delta), (+/delta, 0, +/-delta), (+/delta, +/-delta, 0), (0, +/ -delta, +/-delta)* on (a) control qubits composed of the first set of qubits and the second set of qubits, and (b) the plurality of ancilla qubits to obtain the first hessian quantum circuit block.

**Patentansprüche**

1. Verfahren (200), das von einem System durchgeführt wird, das einen oder mehrere klassische Hardwareprozessoren und eine Mehrzahl von nicht verschränkten Quantenprozessoreinheiten, QPUs, umfasst, wobei der eine oder die mehreren klassischen Hardwareprozessoren durch jeweilige Schnittstellen kommunikativ mit der Mehrzahl von nicht verschränkten QPUs gekoppelt sind, wobei das Verfahren Folgendes umfasst:

Empfangen, über den einen oder die mehreren klassischen Hardwareprozessoren, einer chemischen Verbindung, deren eine oder mehrere Eigenschaften extrahiert werden sollen, wobei die chemische Verbindung mindestens eines von (i) einem Molekül und (ii) einem Feststoff (202) ist;
Erhalten, über den einen oder die mehreren klassischen Hardwareprozessoren, einer Mehrzahl von Atomkoordinaten von jedem einer Mehrzahl von Atomen, die in der chemischen Verbindung vorhanden sind (204);
Bestimmen, über den einen oder die mehreren klassischen Hardwareprozessoren, (i) einer Mehrzahl von Elektronenintegralen, (ii) einer Hamilton-Kernmatrix und (iii) einer Kollokationsmatrix aus der Mehrzahl von Atomkoordinaten von jedem der Mehrzahl von Atomen, die in der chemischen Verbindung vorhanden sind, wobei die Kollokationsmatrix eine rechteckige Matrix der Dimension $N_g$ und $N_{ao}$ ist und wobei $N_g$ eine Anzahl von realen Raumgitterpunkten darstellt, $N_{ao}$ eine Anzahl von Basisfunktionen ist (206);
Bestimmen, über die Mehrzahl von nicht verschränkten QPUs, einer anfänglichen Dichtematrix der chemischen Verbindung aus der Hamilton-Kernmatrix, wobei die anfängliche Dichtematrix unter Verwendung von (i) einer einheitlichen Einzelteilchen-Rotationsmatrix, die durch Diagonalisieren der Hamilton-Kernmatrix erhalten wird, und (ii) Elektronenbelegungen konstruiert wird (208);
iteratives Aktualisieren, über die Mehrzahl von nicht verschränkten QPUs, der anfänglichen Dichtematrix, bis ein Konvergenzkriterium erfüllt ist, um eine endgültige Dichtematrix der chemischen Verbindung zu erhalten (210), wobei das iterative Aktualisieren der anfänglichen Dichtematrix bei jeder Iteration Folgendes umfasst:

(a) Berechnen einer direkten, J, Matrix aus der anfänglichen Dichtematrix (210a);
(b) Bestimmen einer Korrelationsaustauschmatrix basierend auf der anfänglichen Dichtematrix, der Kollokationsmatrix, einem Gradienten der Kollokationsmatrix und einer Hesse der Kollokationsmatrix für meta-generalisierte Gradientenapproximation, meta-GGA, (210b) durch:

(i) Codieren der Kollokationsmatrix auf (a) Steuer-Qubits, die aus einem ersten Satz von Qubits und einem zweiten Satz von Qubits zusammengesetzt sind, und (b) einer Mehrzahl von Ancilla-Qubits in einer zweiten Quantenschaltung, um einen ersten Quantenschaltungsblock zu erhalten, wobei der erste Satz von Qubits mit der Anzahl von Punkten auf dem numerischen Gitter assoziiert ist, der zweite Satz von Qubits mit der Anzahl von Atomorbitalen assoziiert ist und die Mehrzahl von Ancilla-Qubits verwendet wird, um numerische Einträge der Kollokationsmatrix und der anfänglichen Dichtematrix zu speichern (210b1);

(ii) Codieren eines Gradienten der Kollokationsmatrix auf der Quantenschaltung, um einen Gradienten zu erhalten, um einen ersten Gradienten-Quantenschaltungsblock zu erhalten;

(iii) Codieren einer Hesse der Kollokationsmatrix auf der Quantenschaltung, um einen ersten Hesse-Quantenschaltungsblock zu erhalten (210b2);

(iv) Zusammensetzen des ersten Quantenschaltungsblocks mit einem zweiten Quantenschaltungsblock, der eine Zwischendichtematrix auf (a) Steuer-Qubits, die aus dem zweiten Satz von Qubits zusammengesetzt sind, und (b) einem Ancilla-Qubit in der Quantenschaltung codiert, um einen dritten Quantenschaltungsblock zu konstruieren (210b3);

(v) Codieren einer elektronischen Dichte auf der Quantenschaltung durch Durchführen einer sequentiellen Matrixmultiplikation der Kollokationsmatrix, der Dichtematrix und der Kollokationsmatrix, um einen vierten Quantenschaltungsblock zu erhalten (210b4);

(vi) Zusammensetzen des dritten Quantenschaltungsblocks mit dem ersten Gradienten-Quantenschaltungsblock, der den Gradienten der Kollokationsmatrix codiert, um einen zweiten Gradienten-Quantenschaltungsblock zu konstruieren, der einen Gradienten der elektronischen Dichte der chemischen Verbindung codiert (210b5);

(vii) Zusammensetzen des ersten Hesse-Quantenschaltungsblocks, der die Hesse der Kollokationsmatrix codiert, mit dem ersten Quantenschaltungsblock und dem zweiten Quantenschaltungsblock, der die Anfangsdichtematrix sequentiell codiert, um einen zweiten Hesse-Quantenschaltungsblock zu konstruieren, der eine erste Komponente der Hesse der elektronischen Dichte codiert (210b6);

(viii) Zusammensetzen des ersten Hesse-Quantenschaltungsblocks, der die Hesse der Kollokationsmatrix codiert, mit dem zweiten Quantenschaltungsblock, der die Anfangsdichtematrix codiert, und dem ersten Quantenschaltungsblock, der die Kollokationsmatrix sequentiell codiert, um einen dritten Hesse-Quantenschaltungsblock zu konstruieren, der eine zweite Komponente der Hesse der elektronischen Dichte codiert (210b7);

(ix) Zusammensetzen des ersten Hesse-Quantenschaltungsblocks, der die Hesse der Kollokationsmatrix codiert, mit dem zweiten Quantenschaltungsblock, der die Anfangsdichtematrix codiert, und dem ersten Gradienten-Quantenschaltungsblock, der den Gradienten der Kollokationsmatrix codiert, um einen vierten Hesse-Quantenschaltungsblock zu konstruieren, der eine dritte Komponente der Hesse der elektronischen Dichte der chemischen Verbindung codiert (210b8);

(x) Berechnen der Hesse der elektronischen Dichte der chemischen Verbindung durch Addieren der ersten, zweiten und dritten Komponente der Hesse der elektronischen Dichte der chemischen Verbindung unter Verwendung der Mehrzahl von Hilfsqubits (210b9);

(xi) Codieren der Hesse der elektronischen Dichte der chemischen Verbindung auf (a) Steuer-Qubits, die aus einem ersten Satz von Qubits und einem zweiten Satz von Qubits zusammengesetzt sind, und (b) der Mehrzahl von Hilfsqubits, um einen fünften Hesse-Quantenschaltungsblock zu erhalten (210b10);

(xii) Einfügen des vierten Quantenschaltungsblocks mit einem fünften Quantenschaltungsblock für einen phasenwinkelverschobenen Reflektor, des zweiten Gradienten-Quantenschaltungsblocks, der den Gradienten der elektronischen Dichte codiert, und des fünften Hesse-Quantenschaltungsblocks, der die Hesse der elektronischen Dichte codiert, in eine Quantensignalverarbeitungssequenz, um einen sechsten Quantenschaltungsblock zu erzeugen, der eine Chebyshev-Polynomapproximation einer nichtlinearen Funktion der elektronischen Dichte codiert, wobei die nichtlineare Funktion eine Ableitung der elektronischen Energiedichte in Bezug auf die elektronische Dichte ist (210b11);

(xiii) Zusammensetzen des sechsten Quantenschaltungsblocks mit dem ersten Quantenschaltungsblock, um einen siebten Quantenschaltungsblock zu erzeugen, der eine Z-Matrix codiert (210b12); und

(xiv) Zusammensetzen des siebten Quantenschaltungsblocks mit dem ersten Quantenschaltungsblock, um einen Acht-Quantenschaltungsblock zu erzeugen, der die Korrelationsaustauschmatrix codiert (210b13) ;

(c) Bestimmen einer Fock-, F,-Matrix durch Addieren der $J$-Matrix und der Korrelationsaustauschmatrix (210c);

(d) Durchführen einer qubitisierten Diagonalisierung der F-Matrix, um eine Zwischendichtematrix zu erhalten (210d); und

(e) Wiederholen der Schritte (a) bis (d), bis das Konvergenzkriterium erfüllt ist, um die endgültige Dichtematrix der chemischen Verbindung zu erhalten, wobei die Zwischendichtematrix als die anfängliche Dichtematrix betrachtet wird, bis das Konvergenzkriterium erfüllt ist (210e); und

Verwenden, über den einen oder die mehreren klassischen Hardwareprozessoren, der endgültigen Dichtematrix der chemischen Verbindung, um die eine oder die mehreren Eigenschaften der chemischen Verbindung zu extrahieren (212).

2. Verfahren nach Anspruch 1, wobei das Konvergenzkriterium erfüllt ist, wenn eine Norm einer Differenz zwischen der Zwischendichtematrix bei einer aktuellen Iteration und der anfänglichen Dichtematrix bei einer vorherigen Iteration kleiner als ein vordefinierter Schwellenwert ist.

3. Verfahren nach Anspruch 1, wobei die (i) Steuer-Qubits, die aus dem ersten Satz von Qubits und dem zweiten Satz von Qubits bestehen, und (ii) die Mehrzahl von Ancilla-Qubits, um den ersten Quantenschaltungsblock zu erhalten, mathematisch dargestellt werden als: $log\,N_g + log\,N_{ao} + 2\,qubits$, wobei $log\,N_g$ den ersten Satz von Qubits darstellt, $log\,N_{ao}$ den zweiten Satz von Qubits darstellt und 2 Qubits die Mehrzahl von Ancilla-Qubits darstellen.

4. Verfahren nach Anspruch 1, wobei die Hesse der Kollokationsmatrix erhalten wird durch Codieren der Kollokationsmatrix für einen Satz von Realraum-Gitterpunkten $(+/-delta, 0,0)$, $(0, +/-delta, 0)$, $(0,0, +/-delta)$, $(+/\,delta, 0, +/-delta)$, $(+/delta, +/-delta, 0)$, $(0, +/-delta, +/-delta)$ auf (a) Steuer-Qubits, die aus dem ersten Satz von Qubits und dem zweiten Satz von Qubits zusammengesetzt sind, und (b) der Mehrzahl von Ancilla-Qubits, um den ersten Hesse-Quantenschaltungsblock zu erhalten.

5. System (100), das Folgendes umfasst:
einen oder mehrere klassische Hardwareprozessoren (108) und eine Mehrzahl von nicht verschränkten Quantenprozessoren (122), wobei der eine oder die mehreren klassischen Hardwareprozessoren (108) durch jeweilige Schnittstellen kommunikativ mit der Mehrzahl von nicht verschränkten Quantenprozessoren (122) gekoppelt sind, wobei der eine oder die mehreren klassischen Hardwareprozessoren kommunikativ mit mindestens einem Speicher (110) gekoppelt sind, der programmierte Anweisungen speichert; eine oder mehrere Eingabe/Ausgabe-Schnittstellen (I/O-Schnittstellen) (116); und die Mehrzahl von nicht verschränkten Quantenprozessoren (122) operativ mit dem mindestens einen Quantenspeicher (124) gekoppelt sind, wobei der eine oder die mehreren klassischen Hardwareprozessoren (108) und die Mehrzahl von nicht verschränkten Quantenprozessoren (122) durch die programmierten Anweisungen konfiguriert sind zum:

Empfangen, über den einen oder die mehreren klassischen Hardwareprozessoren, einer chemischen Verbindung, deren eine oder mehrere Eigenschaften extrahiert werden sollen, wobei die chemische Verbindung mindestens eines von (i) einem Molekül und (ii) einem Feststoff ist;
Erhalten, über den einen oder die mehreren klassischen Hardwareprozessoren, einer Mehrzahl von Atomkoordinaten von jedem einer Mehrzahl von Atomen, die in der chemischen Verbindung vorhanden sind;
Bestimmen, über den einen oder die mehreren klassischen Hardwareprozessoren, (i) einer Mehrzahl von Elektronenintegralen, (ii) einer Hamilton-Kernmatrix und (iii) einer Kollokationsmatrix aus der Mehrzahl von Atomkoordinaten von jedem der Mehrzahl von Atomen, die in der chemischen Verbindung vorhanden sind, wobei die Kollokationsmatrix eine rechteckige Matrix der Dimension $N_g$ und $N_{ao}$ ist und wobei $N_g$ eine Anzahl von realen Raumgitterpunkten darstellt, $N_{ao}$ eine Anzahl von Basisfunktionen ist;
Bestimmen, über die Mehrzahl von nicht verschränkten Quantenprozessoren, einer anfänglichen Dichtematrix der chemischen Verbindung aus der Hamilton-Kernmatrix, wobei die anfängliche Dichtematrix unter Verwendung von (i) einer einheitlichen Einzelteilchen-Rotationsmatrix, die durch Diagonalisieren der Hamilton-Kernmatrix erhalten wird, und (ii) Elektronenbelegungen konstruiert wird;
iteratives Aktualisieren, über die Mehrzahl von nicht verschränkten Quantenprozessoren, der anfänglichen Dichtematrix, bis ein Konvergenzkriterium erfüllt ist, um eine endgültige Dichtematrix der chemischen Verbindung zu erhalten, wobei das iterative Aktualisieren der anfänglichen Dichtematrix bei jeder Iteration Folgendes umfasst:

(a) Berechnen einer direkten, J, Matrix aus der anfänglichen Dichtematrix;

(b) Bestimmen einer Korrelationsaustauschmatrix basierend auf der anfänglichen Dichtematrix, der Kollokationsmatrix, einem Gradienten der Kollokationsmatrix und einer Hesse der Kollokationsmatrix für meta-generalisierte Gradientenapproximation, meta-GGA, durch:

(i) Codieren der Kollokationsmatrix auf (a) Steuer-Qubits, die aus einem ersten Satz von Qubits und einem zweiten Satz von Qubits zusammengesetzt sind, und (b) einer Mehrzahl von Ancilla-Qubits in einer zweiten Quantenschaltung, um einen ersten Quantenschaltungsblock zu erhalten, wobei der erste Satz von Qubits mit der Anzahl von Punkten auf dem numerischen Gitter assoziiert ist, der zweite Satz von Qubits mit der Anzahl von Atomorbitalen assoziiert ist und die Mehrzahl von Ancilla-Qubits verwendet wird, um numerische Einträge der Kollokationsmatrix und der anfänglichen Dichtematrix zu speichern;

(ii) Codieren eines Gradienten der Kollokationsmatrix auf der Quantenschaltung, um einen Gradienten zu erhalten, um einen ersten Gradienten-Quantenschaltungsblock zu erhalten;

(iii) Codieren einer Hesse der Kollokationsmatrix auf der Quantenschaltung, um einen ersten Hesse-Quantenschaltungsblock zu erhalten;

(iv) Zusammensetzen des ersten Quantenschaltungsblocks mit einem zweiten Quantenschaltungsblock, der eine Zwischendichtematrix auf (a) Steuer-Qubits, die aus dem zweiten Satz von Qubits zusammengesetzt sind, und (b) einem Ancilla-Qubit in der Quantenschaltung codiert, um einen dritten Quantenschaltungsblock zu konstruieren;

(v) Codieren einer elektronischen Dichte auf der Quantenschaltung durch Durchführen einer sequentiellen Matrixmultiplikation der Kollokationsmatrix, der Dichtematrix und der Kollokationsmatrix, um einen vierten Quantenschaltungsblock zu erhalten;

(vi) Zusammensetzen des dritten Quantenschaltungsblocks mit dem ersten Gradienten-Quantenschaltungsblock, der den Gradienten der Kollokationsmatrix codiert, um einen zweiten Gradienten-Quantenschaltungsblock zu konstruieren, der einen Gradienten der elektronischen Dichte der chemischen Verbindung codiert;

(vii) Zusammensetzen des ersten Hesse-Quantenschaltungsblocks, der die Hesse der Kollokationsmatrix codiert, mit dem ersten Quantenschaltungsblock und dem zweiten Quantenschaltungsblock, der die Anfangsdichtematrix sequentiell codiert, um einen zweiten Hesse-Quantenschaltungsblock zu konstruieren, der eine erste Komponente der Hesse der elektronischen Dichte codiert;

(viii) Zusammensetzen des ersten Hesse-Quantenschaltungsblocks, der die Hesse der Kollokationsmatrix codiert, mit dem zweiten Quantenschaltungsblock, der die Anfangsdichtematrix codiert, und dem ersten Quantenschaltungsblock, der die Kollokationsmatrix sequentiell codiert, um einen dritten Hesse-Quantenschaltungsblock zu konstruieren, der eine zweite Komponente der Hesse der elektronischen Dichte codiert;

(ix) Zusammensetzen des ersten Hesse-Quantenschaltungsblocks, der die Hesse der Kollokationsmatrix codiert, mit dem zweiten Quantenschaltungsblock, der die Anfangsdichtematrix codiert, und dem ersten Gradienten-Quantenschaltungsblock, der den Gradienten der Kollokationsmatrix codiert, um einen vierten Hesse-Quantenschaltungsblock zu konstruieren, der eine dritte Komponente der Hesse der elektronischen Dichte der chemischen Verbindung codiert;

(x) Berechnen der Hesse der elektronischen Dichte der chemischen Verbindung durch Addieren der ersten, zweiten und dritten Komponente der Hesse der elektronischen Dichte der chemischen Verbindung unter Verwendung der Mehrzahl von Hilfsqubits;

(xi) Codieren der Hesse der elektronischen Dichte der chemischen Verbindung auf (a) Steuer-Qubits, die aus einem ersten Satz von Qubits und einem zweiten Satz von Qubits zusammengesetzt sind, und (b) der Mehrzahl von Hilfsqubits, um einen fünften Hesse-Quantenschaltungsblock zu erhalten;

(xii) Einfügen des vierten Quantenschaltungsblocks mit einem fünften Quantenschaltungsblock für einen phasenwinkelverschobenen Reflektor, des zweiten Gradienten-Quantenschaltungsblocks, der den Gradienten der elektronischen Dichte codiert, und des fünften Hesse-Quantenschaltungsblocks, der die Hesse der elektronischen Dichte codiert, in eine Quantensignalverarbeitungssequenz, um einen sechsten Quantenschaltungsblock zu erzeugen, der eine Chebyshev-Polynomapproximation einer nichtlinearen Funktion der elektronischen Dichte codiert, wobei die nichtlineare Funktion eine Ableitung der elektronischen Energiedichte in Bezug auf die elektronische Dichte ist;

(xiii) Zusammensetzen des sechsten Quantenschaltungsblocks mit dem ersten Quantenschaltungsblock, um einen siebten Quantenschaltungsblock zu erzeugen, der eine Z-Matrix codiert; und

(xiv) Zusammensetzen des siebten Quantenschaltungsblocks mit dem ersten Quantenschaltungsblock, um einen Acht-Quantenschaltungsblock zu erzeugen, der die Korrelationsaustauschmatrix codiert;

(c) Bestimmen einer Fock-, F,-Matrix durch Addieren der $J$-Matrix und der Korrelationsaustauschmatrix;
(d) Durchführen einer qubitisierten Diagonalisierung der F-Matrix, um eine Zwischendichtematrix zu erhalten; und
(e) Wiederholen der Schritte (a) bis (d), bis das Konvergenzkriterium erfüllt ist, um die endgültige Dichtematrix der chemischen Verbindung zu erhalten, wobei die Zwischendichtematrix als die anfängliche Dichtematrix betrachtet wird, bis das Konvergenzkriterium erfüllt ist; und

Verwenden, über den einen oder die mehreren klassischen Hardwareprozessoren, der endgültigen Dichtematrix der chemischen Verbindung, um die eine oder die mehreren Eigenschaften der chemischen Verbindung zu extrahieren.

6. System nach Anspruch 5, wobei das Konvergenzkriterium erfüllt ist, wenn eine Norm einer Differenz zwischen der Zwischendichtematrix bei einer aktuellen Iteration und der anfänglichen Dichtematrix bei einer vorherigen Iteration kleiner als ein vordefinierter Schwellenwert ist.

7. System nach Anspruch 5, wobei die (i) Steuer-Qubits, die aus dem ersten Satz von Qubits und dem zweiten Satz von Qubits bestehen, und (ii) die Mehrzahl von Ancilla-Qubits, um den ersten Quantenschaltungsblock zu erhalten, mathematisch dargestellt werden als: $log\, N_g + log\, N_{ao} + 2\, qubits$, wobei $log\, N_g$ den ersten Satz von Qubits darstellt, $log\, N_{ao}$ den zweiten Satz von Qubits darstellt und 2 Qubits die Mehrzahl von Ancilla-Qubits darstellen.

8. System nach Anspruch 5, wobei die Hesse der Kollokationsmatrix erhalten wird durch Codieren der Kollokationsmatrix für einen Satz von Realraum-Gitterpunkten $(+/-delta, 0,0)$, $(0, +/-delta, 0)$, $(0,0, +/-delta)$, $(+/delta, 0, +/-delta)$, $(+/delta, +/-delta, 0)$, $(0, +/-delta, +/-delta)$ auf (a) Steuer-Qubits, die aus dem ersten Satz von Qubits und dem zweiten Satz von Qubits zusammengesetzt sind, und (b) der Mehrzahl von Ancilla-Qubits, um den ersten Hesse-Quantenschaltungsblock zu erhalten.

9. Ein oder mehrere nichtflüchtige maschinenlesbare Informationsspeichermedien, die eine oder mehrere Anweisungen umfassen, die, wenn sie von einem oder mehreren klassischen Hardwareprozessoren und einer Mehrzahl von nicht verschränkten Quantenprozessoren, QPUs, ausgeführt werden, Folgendes bewirken:

Empfangen, über den einen oder die mehreren klassischen Hardwareprozessoren, einer chemischen Verbindung, deren eine oder mehrere Eigenschaften extrahiert werden sollen, wobei die chemische Verbindung mindestens eines von (i) einem Molekül und (ii) einem Feststoff ist;
Erhalten, über den einen oder die mehreren klassischen Hardwareprozessoren, einer Mehrzahl von Atomkoordinaten von jedem einer Mehrzahl von Atomen, die in der chemischen Verbindung vorhanden sind;
Bestimmen, über den einen oder die mehreren klassischen Hardwareprozessoren, (i) einer Mehrzahl von Elektronenintegralen, (ii) einer Hamilton-Kernmatrix und (iii) einer Kollokationsmatrix aus der Mehrzahl von Atomkoordinaten von jedem der Mehrzahl von Atomen, die in der chemischen Verbindung vorhanden sind, wobei die Kollokationsmatrix eine rechteckige Matrix der Dimension $N_g$ und $N_{ao}$ ist und wobei $N_g$ eine Anzahl von realen Raumgitterpunkten darstellt, $N_{ao}$ eine Anzahl von Basisfunktionen ist;
Bestimmen, über die Mehrzahl von nicht verschränkten QPUs, einer anfänglichen Dichtematrix der chemischen Verbindung aus der Hamilton-Kernmatrix, wobei die anfängliche Dichtematrix unter Verwendung von (i) einer einheitlichen Einzelteilchen-Rotationsmatrix, die durch Diagonalisieren der Hamilton-Kernmatrix erhalten wird, und (ii) Elektronenbelegungen konstruiert wird;
iteratives Aktualisieren, über die Mehrzahl von nicht verschränkten QPUs, der anfänglichen Dichtematrix, bis ein Konvergenzkriterium erfüllt ist, um eine endgültige Dichtematrix der chemischen Verbindung zu erhalten, wobei das iterative Aktualisieren der anfänglichen Dichtematrix bei jeder Iteration Folgendes umfasst:

(a) Berechnen einer direkten, J, Matrix aus der anfänglichen Dichtematrix;
(b) Bestimmen einer Korrelationsaustauschmatrix basierend auf der anfänglichen Dichtematrix, der Kollokationsmatrix, einem Gradienten der Kollokationsmatrix und einer Hesse der Kollokationsmatrix für meta-generalisierte Gradientenapproximation, meta-GGA, durch:

(i) Codieren der Kollokationsmatrix auf (a) Steuer-Qubits, die aus einem ersten Satz von Qubits und einem zweiten Satz von Qubits zusammengesetzt sind, und (b) einer Mehrzahl von Ancilla-Qubits in einer zweiten Quantenschaltung, um einen ersten Quantenschaltungsblock zu erhalten, wobei der erste Satz von Qubits mit der Anzahl von Punkten auf dem numerischen Gitter assoziiert ist, der zweite Satz von Qubits mit der Anzahl von Atomorbitalen assoziiert ist und die Mehrzahl von Ancilla-Qubits verwendet wird, um numerische Einträge der Kollokationsmatrix und der anfänglichen Dichtematrix zu speichern;

(ii) Codieren eines Gradienten der Kollokationsmatrix auf der Quantenschaltung, um einen Gradienten zu erhalten, um einen ersten Gradienten-Quantenschaltungsblock zu erhalten;

(iii) Codieren einer Hesse der Kollokationsmatrix auf der Quantenschaltung, um einen ersten Hesse-Quantenschaltungsblock zu erhalten;

(iv) Zusammensetzen des ersten Quantenschaltungsblocks mit einem zweiten Quantenschaltungsblock, der eine Zwischendichtematrix auf (a) Steuer-Qubits, die aus dem zweiten Satz von Qubits zusammengesetzt sind, und (b) einem Ancilla-Qubit in der Quantenschaltung codiert, um einen dritten Quantenschaltungsblock zu konstruieren;

(v) Codieren einer elektronischen Dichte auf der Quantenschaltung durch Durchführen einer sequentiellen Matrixmultiplikation der Kollokationsmatrix, der Dichtematrix und der Kollokationsmatrix, um einen vierten Quantenschaltungsblock zu erhalten;

(vi) Zusammensetzen des dritten Quantenschaltungsblocks mit dem ersten Gradienten-Quantenschaltungsblock, der den Gradienten der Kollokationsmatrix codiert, um einen zweiten Gradienten-Quantenschaltungsblock zu konstruieren, der einen Gradienten der elektronischen Dichte der chemischen Verbindung codiert;

(vii) Zusammensetzen des ersten Hesse-Quantenschaltungsblocks, der die Hesse der Kollokationsmatrix codiert, mit dem ersten Quantenschaltungsblock und dem zweiten Quantenschaltungsblock, der die Anfangsdichtematrix sequentiell codiert, um einen zweiten Hesse-Quantenschaltungsblock zu konstruieren, der eine erste Komponente der Hesse der elektronischen Dichte codiert;

(viii) Zusammensetzen des ersten Hesse-Quantenschaltungsblocks, der die Hesse der Kollokationsmatrix codiert, mit dem zweiten Quantenschaltungsblock, der die Anfangsdichtematrix codiert, und dem ersten Quantenschaltungsblock, der die Kollokationsmatrix sequentiell codiert, um einen dritten Hesse-Quantenschaltungsblock zu konstruieren, der eine zweite Komponente der Hesse der elektronischen Dichte codiert;

(ix) Zusammensetzen des ersten Hesse-Quantenschaltungsblocks, der die Hesse der Kollokationsmatrix codiert, mit dem zweiten Quantenschaltungsblock, der die Anfangsdichtematrix codiert, und dem ersten Gradienten-Quantenschaltungsblock, der den Gradienten der Kollokationsmatrix codiert, um einen vierten Hesse-Quantenschaltungsblock zu konstruieren, der eine dritte Komponente der Hesse der elektronischen Dichte der chemischen Verbindung codiert;

(x) Berechnen der Hesse der elektronischen Dichte der chemischen Verbindung durch Addieren der ersten, zweiten und dritten Komponente der Hesse der elektronischen Dichte der chemischen Verbindung unter Verwendung der Mehrzahl von Hilfsqubits;

(xi) Codieren der Hesse der elektronischen Dichte der chemischen Verbindung auf (a) Steuer-Qubits, die aus einem ersten Satz von Qubits und einem zweiten Satz von Qubits zusammengesetzt sind, und (b) der Mehrzahl von Hilfsqubits, um einen fünften Hesse-Quantenschaltungsblock zu erhalten;

(xii) Einfügen des vierten Quantenschaltungsblocks mit einem fünften Quantenschaltungsblock für einen phasenwinkelverschobenen Reflektor, des zweiten Gradienten-Quantenschaltungsblocks, der den Gradienten der elektronischen Dichte codiert, und des fünften Hesse-Quantenschaltungsblocks, der die Hesse der elektronischen Dichte codiert, in eine Quantensignalverarbeitungssequenz, um einen sechsten Quantenschaltungsblock zu erzeugen, der eine Chebyshev-Polynomapproximation einer nichtlinearen Funktion der elektronischen Dichte codiert, wobei die nichtlineare Funktion eine Ableitung der elektronischen Energiedichte in Bezug auf die elektronische Dichte ist;

(xiii) Zusammensetzen des sechsten Quantenschaltungsblocks mit dem ersten Quantenschaltungsblock, um einen siebten Quantenschaltungsblock zu erzeugen, der eine Z-Matrix codiert; und

(xiv) Zusammensetzen des siebten Quantenschaltungsblocks mit dem ersten Quantenschaltungsblock, um einen Acht-Quantenschaltungsblock zu erzeugen, der die Korrelationsaustauschmatrix codiert;

(c) Bestimmen einer Fock-, F,-Matrix durch Addieren der $J$-Matrix und der Korrelationsaustauschmatrix;

(d) Durchführen einer qubitisierten Diagonalisierung der $F$-Matrix, um eine Zwischendichtematrix zu erhalten; und

(e) Wiederholen der Schritte (a) bis (d), bis das Konvergenzkriterium erfüllt ist, um die endgültige Dichtematrix der chemischen Verbindung zu erhalten, wobei die Zwischendichtematrix als die anfängliche Dichtematrix betrachtet wird, bis das Konvergenzkriterium erfüllt ist; und

Verwenden, über den einen oder die mehreren klassischen Hardwareprozessoren, der endgültigen Dichtematrix der chemischen Verbindung, um die eine oder die mehreren Eigenschaften der chemischen Verbindung zu extrahieren.

10. Ein oder mehrere nichtflüchtige maschinenlesbare Informationsspeichermedien nach Anspruch 9, wobei das Konvergenzkriterium erfüllt ist, wenn eine Norm einer Differenz zwischen der Zwischendichtematrix bei einer aktuellen Iteration und der anfänglichen Dichtematrix bei einer vorherigen Iteration kleiner als ein vordefinierter Schwellenwert ist.

11. Ein oder mehrere nichtflüchtige maschinenlesbare Informationsspeichermedien nach Anspruch 9, wobei die (i) Steuer-Qubits, die aus dem ersten Satz von Qubits und dem zweiten Satz von Qubits bestehen, und (ii) die Mehrzahl von Ancilla-Qubits, um den ersten Quantenschaltungsblock zu erhalten, mathematisch dargestellt werden als: *log $N_g$ + log $N_{ao}$ + 2 qubits,* wobei *log $N_g$* den ersten Satz von Qubits darstellt, *log $N_{ao}$* den zweiten Satz von Qubits darstellt und 2 Qubits die Mehrzahl von Ancilla-Qubits darstellen.

12. Ein oder mehrere nichtflüchtige maschinenlesbare Informationsspeichermedien nach Anspruch 9, wobei die Hesse der Kollokationsmatrix erhalten wird durch Codieren der Kollokationsmatrix für einen Satz von Realraum-Gitterpunkten (*+/-delta,* 0,0), (0, *+/ -delta, 0),* (0,0, *+/-delta*), (*+/delta, 0, +/-delta*), (*+/delta, +/ -delta, 0*), (0, *+/-delta, +/-delta*) auf (a) Steuer-Qubits, die aus dem ersten Satz von Qubits und dem zweiten Satz von Qubits zusammengesetzt sind, und (b) der Mehrzahl von Ancilla-Qubits, um den ersten Hesse-Quantenschaltungsblock zu erhalten.

## Revendications

1. Procédé (200), réalisé par un système comprenant un ou plusieurs processeurs matériels classiques et une pluralité d'unités de processeur quantique, QPU, non enchevêtrées, dans lequel les un ou plusieurs processeurs matériels classiques sont couplés de manière communicante à la pluralité de QPU non enchevêtrées par des interfaces respectives, dans lequel le procédé comprend :

la réception, via les un ou plusieurs processeurs matériels classiques, d'un composé chimique dont une ou plusieurs propriétés doivent être extraites, dans lequel le composé chimique est au moins l'un parmi (i) une molécule, et (ii) un solide (202) ;
l'obtention, via les un ou plusieurs processeurs matériels classiques, d'une pluralité de coordonnées atomiques de chacun d'une pluralité d'atomes présents dans le composé chimique (204) ;
la détermination, via les un ou plusieurs processeurs matériels classiques, (i) d'une pluralité d'intégrales électroniques, (ii) d'une matrice hamiltonienne de coeur, et (iii) d'une matrice de colocation, à partir de la pluralité de coordonnées atomiques de chacun de la pluralité d'atomes présents dans le composé chimique, dans lequel la matrice de colocation est une matrice rectangulaire de dimension $N_g$ et $N_{ao}$, et dans lequel $N_g$ représente un nombre de points de grille d'espace réel, et $N_{ao}$ est un nombre de fonctions de base (206) ;
la détermination, via la pluralité de QPU non enchevêtrées, d'une matrice de densité initiale du composé chimique à partir de la matrice hamiltonienne de coeur, dans lequel la matrice de densité initiale est construite en utilisant (i) une matrice de rotation unitaire de particules uniques qui est obtenue en diagonalisant la matrice hamiltonienne de coeur, et (ii) des occupations électroniques (208) ;
la mise à jour itérative, via la pluralité de QPU non enchevêtrées, de la matrice de densité initiale jusqu'à ce qu'un critère de convergence soit satisfait pour obtenir une matrice de densité finale du composé chimique (210), dans lequel la mise à jour itérative de la matrice de densité initiale à chaque itération comprend :

(a) le calcul d'une matrice directe, J, à partir de la matrice de densité initiale (210a) ;
(b) la détermination d'une matrice d'échange de corrélation sur la base de la matrice de densité initiale, de la matrice de colocation, d'un gradient de la matrice de colocation, et d'un hessien de la matrice de colocation pour une approximation de gradient généralisée méta, GGA méta, (210b) par :

(i) le codage de la matrice de colocation sur (a) des qubits de commande composés d'un premier ensemble de qubits et d'un second ensemble de qubits, et (b) d'une pluralité de qubits auxiliaires dans un second circuit quantique, pour obtenir un premier bloc de circuit quantique, dans lequel le premier ensemble de qubits est associé au nombre de points sur la grille numérique, le second ensemble de qubits est associé au nombre d'orbitales atomiques, et la pluralité de qubits auxiliaires sont utilisés pour stocker des entrées numériques de la matrice de colocation et de la matrice de densité initiale (210b1) ;
(ii) le codage d'un gradient de la matrice de colocation sur le circuit quantique pour obtenir un gradient pour obtenir un premier bloc de circuit quantique de gradient ;

(iii) le codage d'un hessien de la matrice de colocation sur le circuit quantique pour obtenir un premier bloc de circuit quantique hessien (210b2);

(iv) la composition du premier bloc de circuit quantique avec un deuxième bloc de circuit quantique qui code une matrice de densité intermédiaire sur (a) des qubits de commande composés du second ensemble de qubits et (b) d'un qubit auxiliaire dans le circuit quantique, pour construire un troisième bloc de circuit quantique (210b3) ;

(v) le codage d'une densité électronique sur le circuit quantique en effectuant une multiplication matricielle séquentielle de la matrice de colocation, de la matrice de densité, et de la matrice de colocation pour obtenir un quatrième bloc de circuit quantique (210b4) ;

(vi) la composition du troisième bloc de circuit quantique avec le premier bloc de circuit quantique de gradient qui a codé le gradient de la matrice de colocation, pour construire un deuxième bloc de circuit quantique de gradient qui code un gradient de la densité électronique du composé chimique (210b5) ;

(vii) la composition du premier bloc de circuit quantique hessien qui code le hessien de la matrice de colocation avec le premier bloc de circuit quantique et le deuxième bloc de circuit quantique qui code la matrice de densité initiale séquentiellement, pour construire un deuxième bloc de circuit quantique hessien qui code une première composante de hessien de la densité électronique (210b6) ;

(viii) la composition du premier bloc de circuit quantique hessien qui code le hessien de la matrice de colocation avec le deuxième bloc de circuit quantique qui code la matrice de densité initiale et le premier bloc de circuit quantique qui code la matrice de colocation séquentiellement, pour construire un troisième bloc de circuit quantique hessien qui code une deuxième composante de hessien de la densité électronique (210b7) ;

(ix) la composition du premier bloc de circuit quantique hessien qui code le hessien de la matrice de colocation avec le deuxième bloc de circuit quantique qui code la matrice de densité initiale et le premier bloc de circuit quantique de gradient qui code le gradient de la matrice de colocation, pour construire un quatrième bloc de circuit quantique hessien qui code une troisième composante de hessien de la densité électronique du composé chimique (210b8) ;

(x) le calcul du hessien de la densité électronique du composé chimique en ajoutant les première, deuxième et troisième composantes de hessien de la densité électronique du composé chimique en utilisant la pluralité de qubits auxiliaires (210b9) ;

(xi) le codage du hessien de la densité électronique du composé chimique sur (a) des qubits de commande composés d'un premier ensemble de qubits et d'un second ensemble de qubits, et (b) la pluralité de qubits auxiliaires, pour obtenir un cinquième bloc de circuit quantique hessien (210b10) ;

(xii) le placement du quatrième bloc de circuit quantique avec un cinquième bloc de circuit quantique pour un réflecteur à déphasage, du deuxième bloc de circuit quantique de gradient qui code le gradient de la densité électronique, et du cinquième bloc de circuit quantique hessien qui code le hessien de la densité électronique dans une séquence de traitement de signal quantique, pour créer un sixième bloc de circuit quantique qui code une approximation polynomiale de Tchebychev d'une fonction non linéaire de la densité électronique, dans lequel la fonction non linéaire est une dérivée de la densité d'énergie électronique par rapport à la densité électronique (210b11) ;

(xiii) la composition du sixième bloc de circuit quantique avec le premier bloc de circuit quantique, pour créer un septième bloc de circuit quantique qui code une matrice Z (210b12) ; et

(xiv) la composition du septième bloc de circuit quantique avec le premier bloc de circuit quantique, pour créer un huitième bloc de circuit quantique qui code la matrice d'échange de corrélation (210b13) ;

(c) la détermination d'une matrice de Fock, F, en ajoutant la matrice $J$ et la matrice d'échange de corrélation (210c) ;

(d) la réalisation d'une diagonalisation qubitisée de la matrice $F$ pour obtenir une matrice de densité intermédiaire (210d) ; et

(e) la répétition des étapes (a) à (d) jusqu'à ce que le critère de convergence soit satisfait pour obtenir la matrice de densité finale du composé chimique, dans lequel la matrice de densité intermédiaire est considérée comme la matrice de densité initiale jusqu'à ce que le critère de convergence soit satisfait (210e) ; et

l'utilisation, via les un ou plusieurs processeurs matériels classiques, de la matrice de densité finale du composé chimique pour extraire les une ou plusieurs propriétés du composé chimique (212).

2. Procédé selon la revendication 1, dans lequel le critère de convergence est satisfait lorsqu'une norme d'une différence entre la matrice de densité intermédiaire à une itération actuelle et la matrice de densité initiale à une itération précédente est inférieure à un seuil prédéfini.

3. Procédé selon la revendication 1, dans lequel les (i) qubits de commande composés du premier ensemble de qubits et du second ensemble de qubits, et (ii) de la pluralité de qubits auxiliaires, pour obtenir le premier bloc de circuit quantique, sont représentés mathématiquement comme : *log $N_g$ + log $N_{ao}$ + 2 qubits,* où *log $N_g$* représente le premier ensemble de qubits, *log $N_{ao}$* représente le second ensemble de qubits, et 2 qubits représente la pluralité de qubits auxiliaires.

4. Procédé selon la revendication 1, dans lequel le hessien de la matrice de colocation est obtenu en codant la matrice de colocation pour un ensemble de points de grille d'espace réel (+/-*delta,* 0,0), *(0, +/-delta, 0),* (0,0, +/-*delta*), (+/*delta, 0, +/-delta),* (+/ *delta, +/-delta,* 0)*,* (0, +/-*delta, +/-delta*) sur (a) des qubits de commande composés du premier ensemble de qubits et du second ensemble de qubits, et (b) la pluralité de qubits auxiliaires pour obtenir le premier bloc de circuit quantique hessien.

5. Système (100), comprenant :
un ou plusieurs processeurs matériels classiques (108) et une pluralité de processeurs quantiques non enchevêtrés (122), dans lequel les un ou plusieurs processeurs matériels classiques (108) sont couplés de manière communicante à la pluralité de processeurs quantiques non enchevêtrés (122) par des interfaces respectives, dans lequel les un ou plusieurs processeurs matériels classiques sont couplés de manière communicante à au moins une mémoire (110) stockant des instructions programmées ; une ou plusieurs interfaces d'entrée/sortie (E/S) (116) ; et la pluralité de processeurs quantiques non enchevêtrés (122) sont couplés de manière opérationnelle à l'au moins une mémoire quantique (124), dans lequel les un ou plusieurs processeurs matériels classiques (108) et la pluralité de processeurs quantiques non enchevêtrés (122) sont configurés par les instructions programmées pour :

recevoir, via les un ou plusieurs processeurs matériels classiques, un composé chimique dont une ou plusieurs propriétés doivent être extraites, dans lequel le composé chimique est au moins l'un parmi (i) une molécule, et (ii) un solide ;
obtenir, via les un ou plusieurs processeurs matériels classiques, une pluralité de coordonnées atomiques de chacun d'une pluralité d'atomes présents dans le composé chimique ;
déterminer, via les un ou plusieurs processeurs matériels classiques, (i) une pluralité d'intégrales électroniques, (ii) une matrice hamiltonienne de coeur, et (iii) une matrice de colocation, à partir de la pluralité de coordonnées atomiques de chacun de la pluralité d'atomes présents dans le composé chimique, dans lequel la matrice de colocation est une matrice rectangulaire de dimension $N_g$ et $N_{ao}$, et dans lequel $N_g$ représente un nombre de points de grille d'espace réel, et $N_{ao}$ est un nombre de fonctions de base ;
déterminer, via la pluralité de processeurs quantiques non enchevêtrés, une matrice de densité initiale du composé chimique à partir de la matrice hamiltonienne de coeur, dans lequel la matrice de densité initiale est construite en utilisant (i) une matrice de rotation unitaire de particules uniques qui est obtenue en diagonalisant la matrice hamiltonienne de coeur, et (ii) des occupations électroniques ;
mettre à jour itérative, via la pluralité de processeurs quantiques non enchevêtrés, la matrice de densité initiale jusqu'à ce qu'un critère de convergence soit satisfait pour obtenir une matrice de densité finale du composé chimique, dans lequel la mise à jour itérative de la matrice de densité initiale à chaque itération comprend :

(a) le calcul d'une matrice directe, J, à partir de la matrice de densité initiale ;
(b) la détermination d'une matrice d'échange de corrélation sur la base de la matrice de densité initiale, de la matrice de colocation, d'un gradient de la matrice de colocation, et d'un hessien de la matrice de colocation pour une approximation de gradient généralisée méta, GGA méta, par :

(i) le codage de la matrice de colocation sur (a) des qubits de commande composés d'un premier ensemble de qubits et d'un second ensemble de qubits, et (b) d'une pluralité de qubits auxiliaires dans un second circuit quantique, pour obtenir un premier bloc de circuit quantique, dans lequel le premier ensemble de qubits est associé au nombre de points sur la grille numérique, le second ensemble de qubits est associé au nombre d'orbitales atomiques, et la pluralité de qubits auxiliaires sont utilisés pour stocker des entrées numériques de la matrice de colocation et de la matrice de densité initiale ;
(ii) le codage d'un gradient de la matrice de colocation sur le circuit quantique pour obtenir un gradient pour obtenir un premier bloc de circuit quantique de gradient ;
(iii) le codage d'un hessien de la matrice de colocation sur le circuit quantique pour obtenir un premier bloc de circuit quantique hessien ;

(iv) la composition du premier bloc de circuit quantique avec un deuxième bloc de circuit quantique qui code une matrice de densité intermédiaire sur (a) des qubits de commande composés du second ensemble de qubits et (b) d'un qubit auxiliaire dans le circuit quantique, pour construire un troisième bloc de circuit quantique ;

(v) le codage d'une densité électronique sur le circuit quantique en effectuant une multiplication matricielle séquentielle de la matrice de colocation, de la matrice de densité, et de la matrice de colocation pour obtenir un quatrième bloc de circuit quantique ;

(vi) la composition du troisième bloc de circuit quantique avec le premier bloc de circuit quantique de gradient qui a codé le gradient de la matrice de colocation, pour construire un deuxième bloc de circuit quantique de gradient qui code un gradient de la densité électronique du composé chimique ;

(vii) la composition du premier bloc de circuit quantique hessien qui code le hessien de la matrice de colocation avec le premier bloc de circuit quantique et le deuxième bloc de circuit quantique qui code la matrice de densité initiale séquentiellement, pour construire un deuxième bloc de circuit quantique hessien qui code une première composante de hessien de la densité électronique ;

(viii) la composition du premier bloc de circuit quantique hessien qui code le hessien de la matrice de colocation avec le deuxième bloc de circuit quantique qui code la matrice de densité initiale et le premier bloc de circuit quantique qui code la matrice de colocation séquentiellement, pour construire un troisième bloc de circuit quantique hessien qui code une deuxième composante de hessien de la densité électronique ;

(ix) la composition du premier bloc de circuit quantique hessien qui code le hessien de la matrice de colocation avec le deuxième bloc de circuit quantique qui code la matrice de densité initiale et le premier bloc de circuit quantique de gradient qui code le gradient de la matrice de colocation, pour construire un quatrième bloc de circuit quantique hessien qui code une troisième composante de hessien de la densité électronique du composé chimique ;

(x) le calcul du hessien de la densité électronique du composé chimique en ajoutant les première, deuxième et troisième composantes de hessien de la densité électronique du composé chimique en utilisant la pluralité de qubits auxiliaires ;

(xi) le codage du hessien de la densité électronique du composé chimique sur (a) des qubits de commande composés d'un premier ensemble de qubits et d'un second ensemble de qubits, et (b) la pluralité de qubits auxiliaires, pour obtenir un cinquième bloc de circuit quantique hessien ;

(xii) le placement du quatrième bloc de circuit quantique avec un cinquième bloc de circuit quantique pour un réflecteur à déphasage, du deuxième bloc de circuit quantique de gradient qui code le gradient de la densité électronique, et du cinquième bloc de circuit quantique hessien qui code le hessien de la densité électronique dans une séquence de traitement de signal quantique, pour créer un sixième bloc de circuit quantique qui code une approximation polynomiale de Tchebychev d'une fonction non linéaire de la densité électronique, dans lequel la fonction non linéaire est une dérivée de la densité d'énergie électronique par rapport à la densité électronique ;

(xiii) la composition du sixième bloc de circuit quantique avec le premier bloc de circuit quantique, pour créer un septième bloc de circuit quantique qui code une matrice Z ; et

(xiv) la composition du septième bloc de circuit quantique avec le premier bloc de circuit quantique, pour créer un huitième bloc de circuit quantique qui code la matrice d'échange de corrélation ;

(c) la détermination d'une matrice de Fock, F, en ajoutant la matrice $J$ et la matrice d'échange de corrélation ;

(d) la réalisation d'une diagonalisation qubitisée de la matrice $F$ pour obtenir une matrice de densité intermédiaire ; et

(e) la répétition des étapes (a) à (d) jusqu'à ce que le critère de convergence soit satisfait pour obtenir la matrice de densité finale du composé chimique, dans lequel la matrice de densité intermédiaire est considérée comme la matrice de densité initiale jusqu'à ce que le critère de convergence soit satisfait ; et

l'utilisation, via les un ou plusieurs processeurs matériels classiques, de la matrice de densité finale du composé chimique pour extraire les une ou plusieurs propriétés du composé chimique.

6. Système selon la revendication 5, dans lequel le critère de convergence est satisfait lorsqu'une norme d'une différence entre la matrice de densité intermédiaire à une itération actuelle et la matrice de densité initiale à une itération précédente est inférieure à un seuil prédéfini.

7. Système selon la revendication 5, dans lequel les (i) qubits de commande composés du premier ensemble de qubits et du second ensemble de qubits, et (ii) de la pluralité de qubits auxiliaires, pour obtenir le premier bloc de circuit quantique, sont représentés mathématiquement comme : *log N$_g$ + log N$_{ao}$ + 2 qubits,* où *log N$_g$* représente le premier ensemble de qubits, *log N$_{ao}$* représente le second ensemble de qubits, et 2 qubits représente la pluralité de qubits auxiliaires.

**8.** Système selon la revendication 5, dans lequel le hessien de la matrice de colocation est obtenu en codant la matrice de colocation pour un ensemble de points de grille d'espace réel *(+/-delta,* 0,0), *(0, +/-delta, 0),* (0,0, *+/-delta),* (+/*delta, 0, +/-delta),* (+/ *delta, +/-delta, 0),* (0, *+/-delta, +/-delta)* sur (a) des qubits de commande composés du premier ensemble de qubits et du second ensemble de qubits, et (b) la pluralité de qubits auxiliaires pour obtenir le premier bloc de circuit quantique hessien.

**9.** Un ou plusieurs supports de stockage d'informations lisibles par machine non transitoires comprenant une ou plusieurs instructions qui, lorsqu'elles sont exécutées par un ou plusieurs processeurs matériels classiques et une pluralité de processeurs quantiques, QPU, non enchevêtrées, amènent :

la réception, via les un ou plusieurs processeurs matériels classiques, d'un composé chimique dont une ou plusieurs propriétés doivent être extraites, dans lequel le composé chimique est au moins l'un parmi (i) une molécule, et (ii) un solide ;

l'obtention, via les un ou plusieurs processeurs matériels classiques, d'une pluralité de coordonnées atomiques de chacun d'une pluralité d'atomes présents dans le composé chimique ;

la détermination, via les un ou plusieurs processeurs matériels classiques, (i) d'une pluralité d'intégrales électroniques, (ii) d'une matrice hamiltonienne de coeur, et (iii) d'une matrice de colocation, à partir de la pluralité de coordonnées atomiques de chacun de la pluralité d'atomes présents dans le composé chimique, dans lequel la matrice de colocation est une matrice rectangulaire de dimension *N$_g$* et *N$_{ao}$,* et dans lequel *N$_g$* représente un nombre de points de grille d'espace réel, et *N$_{ao}$* est un nombre de fonctions de base ;

la détermination, via la pluralité de QPU non enchevêtrées, d'une matrice de densité initiale du composé chimique à partir de la matrice hamiltonienne de coeur, dans lequel la matrice de densité initiale est construite en utilisant (i) une matrice de rotation unitaire de particules uniques qui est obtenue en diagonalisant la matrice hamiltonienne de coeur, et (ii) des occupations électroniques ;

la mise à jour itérative, via la pluralité de QPU non enchevêtrées, de la matrice de densité initiale jusqu'à ce qu'un critère de convergence soit satisfait pour obtenir une matrice de densité finale du composé chimique, dans lequel la mise à jour itérative de la matrice de densité initiale à chaque itération comprend :

(a) le calcul d'une matrice directe, J, à partir de la matrice de densité initiale ;

(b) la détermination d'une matrice d'échange de corrélation sur la base de la matrice de densité initiale, de la matrice de colocation, d'un gradient de la matrice de colocation, et d'un hessien de la matrice de colocation pour une approximation de gradient généralisée méta, GGA méta, par :

(i) le codage de la matrice de colocation sur (a) des qubits de commande composés d'un premier ensemble de qubits et d'un second ensemble de qubits, et (b) d'une pluralité de qubits auxiliaires dans un second circuit quantique, pour obtenir un premier bloc de circuit quantique, dans lequel le premier ensemble de qubits est associé au nombre de points sur la grille numérique, le second ensemble de qubits est associé au nombre d'orbitales atomiques, et la pluralité de qubits auxiliaires sont utilisés pour stocker des entrées numériques de la matrice de colocation et de la matrice de densité initiale ;

(ii) le codage d'un gradient de la matrice de colocation sur le circuit quantique pour obtenir un gradient pour obtenir un premier bloc de circuit quantique de gradient ;

(iii) le codage d'un hessien de la matrice de colocation sur le circuit quantique pour obtenir un premier bloc de circuit quantique hessien ;

(iv) la composition du premier bloc de circuit quantique avec un deuxième bloc de circuit quantique qui code une matrice de densité intermédiaire sur (a) des qubits de commande composés du second ensemble de qubits et (b) d'un qubit auxiliaire dans le circuit quantique, pour construire un troisième bloc de circuit quantique ;

(v) le codage d'une densité électronique sur le circuit quantique en effectuant une multiplication matricielle séquentielle de la matrice de colocation, de la matrice de densité, et de la matrice de colocation pour obtenir un quatrième bloc de circuit quantique ;

(vi) la composition du troisième bloc de circuit quantique avec le premier bloc de circuit quantique de gradient qui a codé le gradient de la matrice de colocation, pour construire un deuxième bloc de circuit quantique de gradient qui code un gradient de la densité électronique du composé chimique ;

(vii) la composition du premier bloc de circuit quantique hessien qui code le hessien de la matrice de colocation avec le premier bloc de circuit quantique et le deuxième bloc de circuit quantique qui code la matrice de densité initiale séquentiellement, pour construire un deuxième bloc de circuit quantique hessien qui code une première composante de hessien de la densité électronique ;

(viii) la composition du premier bloc de circuit quantique hessien qui code le hessien de la matrice de colocation avec le deuxième bloc de circuit quantique qui code la matrice de densité initiale et le premier bloc de circuit quantique qui a codé la matrice de colocation séquentiellement, pour construire un troisième bloc de circuit quantique hessien qui code une deuxième composante de hessien de la densité électronique ;

(ix) la composition du premier bloc de circuit quantique hessien qui code le hessien de la matrice de colocation avec le deuxième bloc de circuit quantique qui code la matrice de densité initiale et le premier bloc de circuit quantique de gradient qui code le gradient de la matrice de colocation, pour construire un quatrième bloc de circuit quantique hessien qui code une troisième composante de hessien de la densité électronique du composé chimique ;

(x) le calcul du hessien de la densité électronique du composé chimique en ajoutant les première, deuxième et troisième composantes de hessien de la densité électronique du composé chimique en utilisant la pluralité de qubits auxiliaires ;

(xi) le codage du hessien de la densité électronique du composé chimique sur (a) des qubits de commande composés d'un premier ensemble de qubits et d'un second ensemble de qubits, et (b) la pluralité de qubits auxiliaires, pour obtenir un cinquième bloc de circuit quantique hessien ;

(xii) le placement du quatrième bloc de circuit quantique avec un cinquième bloc de circuit quantique pour un réflecteur à déphasage, du deuxième bloc de circuit quantique de gradient qui code le gradient de la densité électronique, et du cinquième bloc de circuit quantique hessien qui code le hessien de la densité électronique dans une séquence de traitement de signal quantique, pour créer un sixième bloc de circuit quantique qui code une approximation polynomiale de Tchebychev d'une fonction non linéaire de la densité électronique, dans lequel la fonction non linéaire est une dérivée de la densité d'énergie électronique par rapport à la densité électronique ;

(xiii) la composition du sixième bloc de circuit quantique avec le premier bloc de circuit quantique, pour créer un septième bloc de circuit quantique qui code une matrice $Z$ ; et

(xiv) la composition du septième bloc de circuit quantique avec le premier bloc de circuit quantique, pour créer un huitième bloc de circuit quantique qui code la matrice d'échange de corrélation ;

(c) la détermination d'une matrice Fock, F, en ajoutant la matrice $J$ et la matrice d'échange de corrélation ;

(d) la réalisation d'une diagonalisation qubitisée de la matrice $F$ pour obtenir une matrice de densité intermédiaire ; et

(e) la répétition des étapes (a) à (d) jusqu'à ce que le critère de convergence soit satisfait pour obtenir la matrice de densité finale du composé chimique, dans lequel la matrice de densité intermédiaire est considérée comme la matrice de densité initiale jusqu'à ce que le critère de convergence soit satisfait ; et

l'utilisation, via les un ou plusieurs processeurs matériels classiques, de la matrice de densité finale du composé chimique pour extraire les une ou plusieurs propriétés du composé chimique.

**10.** Un ou plusieurs supports de stockage d'informations lisibles par machine non transitoires selon la revendication 9, dans lequel le critère de convergence est satisfait lorsqu'une norme d'une différence entre la matrice de densité intermédiaire à une itération actuelle et la matrice de densité initiale à une itération précédente est inférieure à un seuil prédéfini.

**11.** Un ou plusieurs supports de stockage d'informations lisibles par machine non transitoires selon la revendication 9, dans lequel les (i) qubits de commande composés du premier ensemble de qubits et du second ensemble de qubits, et (ii) de la pluralité de qubits auxiliaires, pour obtenir le premier bloc de circuit quantique, sont représentés mathématiquement comme : $log\ N_g + log\ N_{ao} + 2\ qubits$, où $log\ N_g$ représente le premier ensemble de qubits, $log\ N_{ao}$ représente le second ensemble de qubits, et 2 qubits représente la pluralité de qubits auxiliaires.

**12.** Un ou plusieurs supports de stockage d'informations lisibles par machine non transitoires selon la revendication 9, dans lequel le hessien de la matrice de colocation est obtenu en codant la matrice de colocation pour un ensemble de points de grille d'espace réel (+/-*delta*, 0,0), (0, +/-*delta*, 0), (0,0, +/-*delta*), (+/*delta*, 0, +/-*delta*), (+/ *delta*, +/-*delta*, 0), (0, +/-*delta*, +/-*delta*) sur (a) des qubits de commande composés du premier ensemble de qubits et du second ensemble de qubits, et (b) la pluralité de qubits auxiliaires pour obtenir le premier bloc de circuit quantique hessien.

100

102

112

MEMORY 110

REPOSITORY 114

I/O INTERFACE
116

CLASSICAL
HARDWARE
PROCESSORS
108

106

104

CONTROL SYSTEM
118

SIGNAL DELIVERY
SYSTEM 120

QUANTUM
PROCESSING UNITS
122

QUANTUM
MEMORY 124

FIG. 1

200

Receive a chemical compound whose one or more properties to be extracted, wherein the chemical compound is at least one of (i) a molecule, and (ii) a solid — 202

obtain a plurality of atomic coordinates of each of a plurality of atoms present in the chemical compound — 204

Determine (i) a plurality of electron integrals, (ii) a core Hamiltonian matrix, and (iii) a collocation matrix, from the plurality of atomic coordinates of each of the plurality of atoms present in the chemical compound, wherein the collocation matrix is a rectangular matrix of dimension $N_g$ and $N_{ao}$, and wherein $N_g$ represents a number of real space grid points, $N_{ao}$ is a number of basis functions — 206

Determine an initial density matrix of the chemical compound from the core Hamiltonian matrix, wherein the initial density matrix is constructed using (i) a single particles unitary rotation matrix which is obtained by diagonalizing the core Hamiltonian matrix, and (ii) electron occupancies — 208

A

FIG. 2A

A

200

iteratively update the initial density matrix until a convergence criteria is satisfied to obtain a final density matrix of the chemical compound (210), wherein iteratively updating the initial density matrix at each iteration comprises: — 210

Computing a direct matrix from the initial density matrix — 210a

Determining a correlation exchange matrix based on the initial density matrix, the collocation matrix, a Gradient of collocation matrix, and a Hessian of the collocation matrix for meta Generalized Gradient Approximation (meta GGA) by: — 210b

Encoding the collocation matrix on (a) control qubits composed of a first set of qubits and a second set of qubits, and (b) a plurality of ancilla qubits in a second quantum circuit, to obtain a first quantum circuit block, wherein the first set of qubits is associated with the number of points on the numerical grid, the second set of qubits is associated with the number of atomic orbitals, and the plurality of ancilla qubits are used to store numerical entries of the collocation matrix and the initial density matrix — 210b1

Encoding a gradient of the collocation matrix on the quantum circuit to obtain a gradient to obtain a first gradient quantum circuit block — 210b2

Encoding a hessian of the collocation matrix on the quantum circuit to obtain a first hessian quantum circuit block — 210b3

B

FIG. 2B

B

210b

Composing the first quantum circuit block with a second quantum circuit block that encodes an intermediate density matrix on (a) control qubits composed of the second set of qubits and (b) an ancilla qubit in the quantum circuit to construct a third quantum circuit block

— 210b4

Encoding an electronic density on the quantum circuit by performing a sequential matrix multiplication of the collocation matrix, the density matrix, and the collocation matrix to obtain a fourth quantum circuit block

— 210b5

Composing the third quantum circuit block with the first gradient quantum circuit block that encoded the gradient of the collocation matrix, to construct a second gradient quantum circuit block that encodes a gradient of the electronic density of the chemical compound

— 210b6

Composing the first hessian quantum circuit block that encodes the hessian of the collocation matrix with the first quantum circuit block and the second quantum circuit block that encodes the initial density matrix sequentially, to construct a second hessian quantum circuit block that encodes a first component of hessian of the electronic density

— 210b7

Composing the first hessian quantum circuit block that encodes the hessian of the collocation matrix with the second quantum circuit block that encodes the initial density matrix and the first quantum circuit block that encoded the collocation matrix sequentially, to construct a third hessian quantum circuit block that encodes a second component of hessian of the electronic density

— 210b8

C

FIG. 2C

C

Composing the first hessian quantum circuit block that encodes the hessian of the collocation matrix with the second quantum circuit block that encodes the initial density matrix and the first gradient quantum circuit block encodes the gradient of the collocation matrix, to construct a fourth hessian quantum circuit block that encodes a third component of hessian of the electronic density of the chemical compound

210b9

computing the hessian of the electronic density of the chemical compound by adding the first, second and third component of hessian of the electronic density of the chemical compound using the plurality of ancilla qubits

210b10

210b11

Encoding the Hessian of the electronic density of the chemical compound on (a) control qubits composed of a first set of qubits and a second set of qubits, and (b) the plurality of ancilla qubits, to obtain a fifth hessian quantum circuit block

Putting the fourth quantum circuit block with a fifth quantum circuit block for a phase angle shifted reflector, the second gradient quantum circuit block that encodes the gradient of the electronic density, and the fifth hessian quantum circuit block that encodes the hessian of the electronic density into a quantum signal processing sequence, to create a sixth quantum circuit block that encodes a Chebyshev polynomial approximation of a nonlinear function of the electronic density, wherein the nonlinear function is a derivative of the electronic energy density with respect to the electronic density

210b12

210b

D

FIG. 2D

200

D

210

Composing the sixth quantum circuit block with the first quantum circuit block, to create a seventh quantum circuit block that encodes a Z matrix

210b11

composing the seventh quantum circuit block with the first quantum circuit block, to create an eight quantum circuit block that encodes the correlation exchange matrix density with respect to the electronic density

210b12

Computing a Fock matrix by adding the direct matrix and the correlation exchange matrix

210c

Performing qubitized diagonalization of the Fock matrix to obtain an updated density matrix, wherein the updated density matrix is used in a subsequent iteration

210d

repeating the steps of computing the direct (J) matrix till performing the qubitized diagonalization of the F matrix until the convergence criteria is satisfied to obtain the final density matrix of the chemical compound, wherein the intermediate density matrix is considered as the initial density matrix until the convergence criteria is satisfied

210e

Utilizing the final density matrix of the chemical compound, to extract one or more properties of the chemical compound

212

FIG. 2E

Input: atomic coordinates of each atom in a chemical compound whose desired properties must be extracted

↓

Determine electron integrals, a core Hamiltonian, and a collocation matrix

↓

Compute initial density matrix (DM) by diagonalizing the core Hamiltonian

↓

Compute direct matrix based on DM

↓

Determine correlation exchange matrix based on the direct matrix and the collocation matrix

↓

Compute a Fock matrix by adding the direct matrix and the correlation exchange matrix

↓

Perform qubitized diagonalization of the Fock matrix to obtain updated DM

↓

Is convergence criteria satisfied?

No → DM = updated DM

Yes ↓

Final DM = updated initial DM

↓

Extract desired properties of the chemical compound using final DM

FIG. 3

FIG. 4

FIG. 5

FIG. 6A

FIG. 6B

FIG. 6C

FIG. 6D

FIG. 6E

FIG. 6F

FIG. 7

FIG. 8

FIG. 9

**EP 4 575 930 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 20232106141

**Non-patent literature cited in the description**

- **ROSSMANNEK MAX et al.** *Quantum HF/DFT-embedding algorithms for electronic structure calculations: Scaling up to complex molecular systems*
- **KO TAEHEE et al.** *Implementation of the Density-functional Theory on Quantum Computers with Linear Scaling with respect to the Number of Atoms*
- **ROBERT SCHADE et al.** *Parallel Quantum Chemistry on Noisy Intermediate-Scale Quantum Computers*
- **ROSSMANNEK MAX et al.** *Quantum Embedding Method for the Simulation of Strongly Correlated Systems on Quantum Computers*
- **SAMBIT DAS et al.** *DFT-FE 1.0: A massively parallel hybrid CPU-GPU density functional theory code using finite-element discretization*
- **RYAN PEDERSON et al.** *Large scale quantum chemistry with Tensor Processing Units*